# EUROPEAN PATENT SPECIFICATION

(11) **EP 1 551 284 B1**
(45) Date of publication and mention of the grant of the patent: **06.04.2016**
(21) Application number: 03776272.1
(22) Date of filing: 10.10.2003
(51) Int. Cl.: A61B 5/00

(54) **NON-INVASIVELY MEASURING HEMODYNAMIC PARAMETERS**
NICHTINVASIVE MESSUNG VON HÄMODYNAMISCHEN PARAMETERN
MESURE NON INVASIVE DE PARAMETRES HEMODYNAMIQUES

(30) Priority: 11.10.2002 US 269801
(43) Date of publication of application: 13.07.2005
(73) Proprietor: Tensys Medical, Inc., San Diego, CA 92121 (US)
(72) Inventor: HESSEL, Stephen, R., San Diego, CA 92131 (US); FINBURGH, Simon, E., San Diego, CA 92122 (US); HEMPSTEAD, Russell, D., Lafayette, CO 80026 (US); PERONA, Mark, W., San Diego, CA 92129 (US); VIDISCHAK, Ronald, J., Escondido, CA 92027 (US); VOSS, Gregory, I., Solana Beach, CA 92075 (US); CONERO, Ronald, S., San Diego, CA 92131 (US); MARKLE, William, H., Laguna Niguel, CA 92677 (US)
(74) Representative: Dolleymores
(86) International application number: PCT/US2003/032132
(87) International publication number: WO 2004/032748

(56) References cited:
- WO-A-01/00087
- US-A- 3 704 708
- US-A- 4 993 422
- US-A- 4 998 534
- US-A- 5 313 952
- US-A- 5 642 733
- US-A- 6 132 383
- US-A1- 2003 153 824
- US-B1- 6 176 831
- US-B1- 6 176 831
- US-B1- 6 241 679
- US-B1- 6 290 650

## Description

### Background of the Invention

### 1. Field of the Invention

This invention relates generally to apparatus and methods for monitoring parameters associated with the circulatory system of a living subject, and specifically to the non-invasive monitoring of arterial blood pressure.

### 2. Description of Related Technology

The accurate, continuous, non-invasive measurement of blood pressure has long been sought by medical science. The availability of such measurement techniques would allow the caregiver to continuously monitor a subject's blood pressure accurately and in repeatable fashion without the use of invasive arterial catheters (commonly known as "A-lines") in any number of settings including, for example, surgical operating rooms where continuous, accurate indications of true blood pressure are often essential.

Several well known techniques have heretofore been used to non-invasively monitor a subject's arterial blood pressure waveform, namely, auscultation, oscillometry, and tonometry. Both the auscultation and oscillometry techniques use a standard inflatable arm cuff that occludes the subject's brachial artery. The auscultatory technique determines the subject's systolic and diastolic pressures by monitoring certain Korotkoff sounds that occur as the cuff is slowly deflated. The oscillometric technique, on the other hand, determines these pressures, as well as the subject's mean pressure, by measuring actual pressure changes that occur in the cuff as the cuff is deflated. Both techniques determine pressure values only intermittently, because of the need to alternately inflate and deflate the cuff, and they cannot replicate the subject's actual blood pressure waveform. Thus, true continuous, beat-to-beat blood pressure monitoring cannot be achieved using these techniques.

Occlusive cuff instruments of the kind described briefly above have generally been somewhat effective in sensing long-term trends in a subject's blood pressure. However, such instruments generally have been ineffective in sensing short-term blood pressure variations, which are of critical importance in many medical applications, including surgery.

The technique of arterial tonometry is also well known in the medical arts. According to the theory of arterial tonometry, the pressure in a superficial artery with sufficient bony support, such as the radial artery, may be accurately recorded during an applanation sweep when the transmural pressure equals zero. The term "applanation" refers generally to the process of varying the pressure applied to the artery. An applanation sweep refers to a time period during which pressure over the artery is varied from overcompression to undercompression or vice versa. At the onset of a decreasing applanation sweep, the artery is overcompressed into a "dog bone" shape, so that pressure pulses are not recorded. At the end of the sweep, the artery is undercompressed, so that minimum amplitude pressure pulses are recorded. Within the sweep, it is assumed that an applanation occurs during which the arterial wall tension is parallel to the tonometer surface. Here, the arterial pressure is perpendicular to the surface and is the only stress detected by the tonometer sensor. At this pressure, it is assumed that the maximum peak-to-peak amplitude (the "maximum pulsatile") pressure obtained corresponds to zero transmural pressure.

Exemplary prior art tonometric approaches are described in for example US Patent No. 6,176,831 to Voss et al, US Patent No. 6,132,383 to Chesney et al, WIPO Publication WO 01/00087 to Baura, US Patent No. 6,290,650 to Butterfield et al, and US Patent No 5,642,733 to Archibald et al, which generally describe single pressure sensor tonometric systems.

Yet another prior art approach for implementing the tonometry technique includes a rigid array of miniature pressure transducers that is applied against the tissue overlying a peripheral artery, e.g., the radial artery. The transducers each directly sense the mechanical forces in the underlying subject tissue, and each is sized to cover only a fraction of the underlying artery. The array is urged against the tissue, to applanate the underlying artery and thereby cause beat-to-beat pressure variations within the artery to be coupled through the tissue to at least some of the transducers. An array of different transducers is used to ensure that at least one transducer is always over the artery, regardless of array position on the subject. This type of tonometer, however, is subject to several drawbacks. First, the array of discrete transducers generally is not anatomically compatible with the continuous contours of the subject's tissue overlying the artery being sensed. This has historically led to inaccuracies in the resulting transducer signals. In addition, in some cases, this incompatibility can cause tissue injury and nerve damage and can restrict blood flow to distal tissue.

Additional prior art approaches include eg US Patent No 5,313,952 to Hoch, US Patent Application Publication NO 2003/0153824 to Tsubata and US Patent No 3,704,708 to Iberall.

Other prior art techniques have sought to more accurately place a single tonometric sensor laterally above the artery, thereby more completely coupling the sensor to the pressure variations within the artery. However, such systems may place the sensor at a location where it is geometrically "centered" but not optimally positioned for signal coupling, and further typically require comparatively frequent re-calibration or repositioning due to movement of the subject during measurement. Additionally, the methodology for proper initial and follow-on placement is awkward, essentially relying on the caregiver to manually locate the optimal location for sensor placement on the subject each time, and then mark that location (such as by keeping their finger on the spot, or alternatively marking it with a pen or other marking instrument), after which the sensor is placed over the mark.

Tonometry systems are also commonly quite sensitive to the orientation of the pressure transducer on the subject being monitored. Specifically, such systems show a degradation in accuracy when the angular relationship between the transducer and the artery is varied from an "optimal" incidence angle. This is an important consideration, since no two measurements are likely to have the device placed or maintained at precisely the same angle with respect to the artery. Many of the foregoing approaches similarly suffer from not being able to maintain a constant angular relationship with the artery regardless of lateral position, due in many cases to positioning mechanisms which are not adapted to account for the anatomic features of the subject, such as curvature of the wrist surface.

Another deficiency of prior art non-invasive hemodynamic measurement technology relates to the lack of disposability of components associated with the device. Specifically, it is desirable to make portions of the device which may (i) be contaminated in any fashion through direct or indirect contact with the subject(s) being monitored); (ii) be specifically calibrated or adapted for use on that subject; (iii) lose calibration through normal use, thereby necessitating a more involved recalibration process (as opposed to simply replacing the component with an unused, calibrated counterpart), or (iv) disposable after one or a limited number of uses. This feature is often frustrated in prior art systems based on a lack of easy replacement of certain components (i.e., the components were not made replaceable during the design process), or a prohibitively high cost associated with replacing components that are replaceable. Ideally, certain components associated with a non-invasive hemodynamic assessment device would be readily disposable and replaced at a very low cost to the operator.

Yet another disability of the prior art concerns the ability to conduct multiple hemodynamic measurements on a subject at different times and/or different locations. For example, where blood pressure measurements are required in first and second locations (e.g., the operating room and recovery room of a hospital), prior art methodologies necessitate either (i) the use of an invasive catheter (A-line), (ii) transport of the entire blood pressure monitoring system between the locations, or (iii) disconnection of the subject at the first monitoring location, transport, and then subsequent connection to a second blood pressure monitoring system at the second location.

The disabilities associated with invasive catheters are well understood. These include the need to perforate the subject's skin (with attendant risk of infection), and discomfort to the subject.

Transport of the entire blood pressure monitoring system is largely untenable, due to the bulk of the system and the desire to maintain monitoring equipment indigenous to specific locations.

Disconnection and subsequent reconnection of the subject is also undesirable, since it requires placing a sensor or apparatus on the patient's anatomy a second time, thereby necessitating recalibration, and reducing the level of confidence that the measurements taken at the two different locations are in fact directly comparable to one another. Specifically, since the sensor and supporting apparatus is physically withdrawn at the first location, and then a new sensor subsequently placed again on the subject's tissue at the second location, the likelihood of having different coupling between the sensor and the underlying blood vessel at the two locations is significant. Hence, identical intra-vascular pressure values may be reflected as two different values at the different locations due to changes in coupling, calibration, sensor parameters, and related factors, thereby reducing the repeatability and confidence level associated the two readings.

Another disability of the prior art relates to the lack of any readily implemented and reliable means or mechanism for correction of blood pressure readings for differences in hydrostatic pressure resulting from differences in elevation between the pressure sensor and the organ of interest. For example, where a surgeon or health care provider wishes to know the actual pressure in the brain or head of the subject, the pressure reading obtained from another location of the body (e.g., the radial artery) must be corrected for the fact that the subject's blood volume exerts additional pressure at the radial artery, presumed to be lower in elevation than the subject's head. The additional pressure is the result of the hydrostatic pressure associated with the equivalent of a "column" of blood existing between the radial artery and the uppermost portions of the subject's anatomy.

Additionally, differences in pressure resulting from hydrodynamic effects associated with the cardiovascular system. While quite complex and sophisticated, the circulatory system of a living being is in effect a piping system which, *inter alia,* generates flow resistance and therefore head loss (pressure drop) as a function of the blood flow there through. Hence, significant difference between the pressures measured at the output of the heart and the radial artery may exist due to purely hydrodynamic effects.

Prior art techniques for correcting for hydrostatic pressure difference generally comprise measuring the difference in elevation between the measurement location and the organ of interest, and then performing a manual or hand calculation of the hydrostatic pressure correction resulting from this difference, based on an assumed gravitational field vector magnitude g (commonly rounded to 9.8 m/s²). Such techniques are cumbersome at best, and prone to significant errors at worst.

Based on the foregoing, there is needed an improved apparatus and methodology for accurately, continuously, and non-invasively measuring blood pressure within a living subject. Such improved apparatus and methodology would ideally allow for prompt and accurate initial placement of the tonometric sensor(s), while also providing robustness and repeatability of placement under varying patient physiology and environmental conditions. Such apparatus would also incorporate low cost and disposable components, which could be readily replaced in the event of contamination or loss of calibration/performance (or purely on a preventive or periodic basis).

Such apparatus and methods would furthermore be easily utilized and maintained by both trained medical personnel and untrained individuals, thereby allowing certain subjects to accurately and reliably conduct self-monitoring and maintenance of the system.

Additionally, the improved apparatus and methods would allow the user or caregiver to readily and accurately correct for hydrostatic and/or hydrodynamic effects associated with hemodynamic parameter measurements.

### Summary of the Invention

The present invention satisfies the aforementioned needs by an improved apparatus according to claim 1 for non-invasively and continuously assessing hemodynamic properties, including arterial blood pressure, within a living subject.

In a further exanmple an improved hemodynamic assessment apparatus is disclosed. The apparatus generally comprises a brace adapted to receive a portion of the anatomy of a living subject; actuator apparatus coupled to the brace and adapted to move a sensor; and alignment apparatus adapted to mate with a portion of the anatomy, the alignment apparatus configured to maintain a desired orientation of the sensor prior to coupling thereof to the actuator. In one exemplary embodiment, the apparatus is adapted to receive the wrist/forearm area of a human being, and the alignment apparatus is configured to position the sensor over the lateral portion of the wrist (i.e., radial artery).

In a further example, an apparatus adapted for a plurality of hemodynamic measurements of a living subject is disclosed. The apparatus generally comprises: an alignment member adapted for removable mating with the anatomy of the subject, the alignment member being configured to maintain a sensor substantially in a desired orientation with respect to said anatomy between the measurements when the sensor is not otherwise positioned by another device. In one exemplary embodiment, the alignment member comprises a molded frame which is adhesively mated to the subject's tissue. The sensor is suspended within the frame such that the sensor can move somewhat with respect to the frame when coupled to a sensor actuator, yet the sensor is captured within a central region of the frame when the sensor is uncoupled from the actuator.

In a further example, an apparatus adapted to position at least one sensor with respect to an anatomy is disclosed. The apparatus generally comprises: alignment apparatus adapted to substantially conform to said anatomy; and positioning apparatus adapted to maintain a substantially fixed position with respect to said anatomy, and cooperate with said alignment apparatus to position said at least one sensor in the desired orientation. In one exemplary embodiment, the alignment apparatus comprises a frame element with removable reticle, and the positioning apparatus comprises an adjustable arm associated with a brace. The positioning arm couples to the frame element in order to maintain a substantially constant relationship between the arm and frame, and hence between the arm and sensor.

In a further example, an improved sensor interface apparatus is disclosed. The interface apparatus generally comprises: a substantially flexible substrate having first and second regions; a data storage element disposed at the first region; a sensor element disposed at the second region; and a plurality of electrically conductive traces disposed at least partially on the substrate, the traces providing electrical continuity between the data storage element and the sensor element. In one exemplary embodiment, the interface has an EEPROM at the first region and a pressure transducer at the second region. The EEPROM end (first region) further includes a plurality of contacts and is adapted to mate with corresponding contacts of a receptacle formed in the actuator housing.

In a further example, an improved hemodynamic assessment apparatus is disclosed. The apparatus generally comprises: an alignment apparatus; and a coupling element cooperating with the alignment apparatus and a sensor to initially position the sensor with respect to the anatomical portion; wherein said coupling element is adapted to be removable from said assessment apparatus to permit optional variable positioning of said sensor subsequent to the initial positioning. In one exemplary embodiment, the alignment apparatus comprises a frame with the sensor suspended within the frame via a flexible sheet or membrane. The coupling element comprises a molded paddle which cooperates with both the sensor and the frame to maintain the sensor in a desired position until the paddle is removed, at which point the sensor is substantially free to move within the frame, e.g., under the action of the actuator mechanism.

In a further example, an improved method of positioning a sensor with respect to the anatomy of a subject is disclosed. The method generally comprises: disposing a marker on a location of the anatomy; disposing the sensor relative to said marker; displacing the marker from said location; and disposing said sensor at said location. In one exemplary embodiment, the marker comprises a reticle which is removably attached to an adhesive element, the adhesive element attached to a frame element in a known relationship (e.g., hinged). A sensor is suspended within the frame element as previously described. The adhesive element is placed on the subject's skin with the reticle aligned over a blood vessel, the reticle removed, and then the frame element (and sensor) swung into place atop the blood vessel and secured in place. A semi-permanent positional relationship between the sensor and blood vessel is therefore established.

In a further example, an improved anatomical sensor alignment apparatus is disclosed. The apparatus generally comprises: a first support element; a marker movably coupled to the first support element; and a second support element disposed in known relationship to said marker. In one exemplary embodiment, the second support element is adapted to receive a sensor; wherein the second support element is movably coupled to the first support element such that the sensor is disposed in a known relationship (e.g., via hinge, or similar mechanical coupling) to the marker when the movable coupling is actuated.

In a further example, an improved blood pressure monitoring system is disclosed. The system generally comprises: at least one pressure sensor adapted to measure a pressure waveform from a blood vessel; an actuator adapted to control the position of the at least one sensor relative to the blood vessel; and a brace adapted to maintain the actuator in a substantially constant position with respect to the blood vessel; wherein the brace is further adapted to maintain the sensor in a desired location prior to coupling of the actuator to the sensor. In one exemplary embodiment, a removable alignment apparatus adapted to maintain said sensor in a desired location prior to coupling of said actuator to said sensor (such as that previously described) is also provided.

In a further example, an improved tonometric pressure sensor apparatus is disclosed. The sensor apparatus generally comprises: a pressure sensor adapted to generate an electrical signal relating to the pressure applied to at least one surface thereof; a housing element adapted to at least partly receive the sensor therein; and a bias element coupled to the housing and adapted to bias tissue of a subject proximate the at least one surface when the apparatus is disposed in contact therewith; wherein the housing element further comprises a coupling adapter for coupling the sensor apparatus to a parent device. In an exemplary embodiment, bias element comprises a foam pad, and the parent device comprises an actuator. The sensor apparatus is further adapted to be retained in a desired position above said blood vessel (when uncoupled from the actuator) via the previously referenced alignment apparatus.

In a further example an improved method of recurrently measuring the blood pressure of a living subject is disclosed. The method generally comprises: disposing an alignment apparatus adapted to align at least one sensor with respect to the anatomy of the subject; positioning the at least one sensor with respect to the anatomy using at least in part the alignment apparatus; measuring blood pressure at a first time using the sensor; and measuring blood pressure at a second time using the sensor, wherein the sensor position is maintained with respect to the anatomy between measurements using the alignment apparatus.

In a further example, an improved apparatus for coupling a movable sensor having a sensing surface to an actuator is disclosed. The coupling apparatus generally comprises: a first coupling element disposed on the sensor; and a second coupling element disposed on the actuator, the second element adapted to receive at least a portion of the first element, thereby coupling the actuator and sensor in a substantially rigid configuration. In one example the first and second coupling elements are substantially pyramid-shaped and inverse pyramid-shaped, respectively, so as to facilitate coupling under conditions where the sensor (and first element) is misaligned with the second element, in both planar ("XY") and rotational dimensions. This arrangement also advantageously provides significant rigidity and lack of compliance between the sensor assembly and actuator when the first and second elements are coupled.

In a further example, an improved sensor support apparatus is disclosed. The apparatus generally comprises: a brace adapted to receive a portion of the anatomy of a subject; and a support member adjustably coupled to the brace, the support member being adapted to position a sensor assembly relative to the portion; wherein the adjustable coupling comprises a ratchet mechanism. In one example the brace comprises a substantially unitary component adapted to support the exterior surfaces of the wrist and forearm of a human, with the ratchet mechanism disposed substantially within the brace.

In a further example, an improved apparatus for controlling the position of a hemodynamic sensor with respect to a subject is disclosed, wherein a single adjustment element permits adjustment of at least three degrees of freedom of the sensor. In one exemplary embodiment, the apparatus comprises a manually adjusted mechanism having an adjusting knob which, when actuated, permits simultaneous movement in five degrees of freedom.

In a further example an improved method of providing treatment to a subject using the aforementioned apparatus and methodologies is disclosed. In one example the method comprises: selecting a blood vessel of the subject useful for measuring hemodynamic data; disposing a marker on a location of the anatomy proximate the blood vessel; disposing the sensor relative to said marker; displacing the marker from said location; disposing said sensor at said location; measuring at least one hemodynamic parameter using the sensor; and providing treatment to the subject based on the hemodynamic data. In a second example the method comprises: selecting a blood vessel of the subject useful for measuring data; disposing an alignment apparatus adapted to align at least one sensor with respect to the blood vessel; positioning the at least one sensor with respect to the blood vessel using at least in part the alignment apparatus; measuring at least one hemodynamic parameter at a first time using the sensor; and measuring the at least one hemodynamic parameter at a second time using the sensor, wherein the sensor position is maintained with respect to the blood vessel between measurements using the alignment apparatus; and providing treatment to the subject based at least in part on the measurements taken at the first and second times.

In a further example, an improved apparatus and methods for displaying and applying hydrostatic and/or hydrodynamic correction factors to hemodynamic parameter measurements are disclosed.

These and other features of the invention will become apparent from the following description of the invention, taken in conjunction with the accompanying drawings.

### Brief Description of the Drawings

Fig. 1 is a perspective view of one exemplary embodiment of the hemodynamic assessment apparatus of the present invention, shown assembled.
Fig. 1a is a top perspective view of one exemplary embodiment of the sensor assembly of the present invention.
Fig. 1b is a cross-sectional view of the sensor assembly of Fig. 1a, taken along line 1b-1b.
Fig. 1c is a cross-sectional view of the sensor assembly of Fig. 1a, taken along line 1c-1c.
Fig. 1d is a top plan view of the apparatus of Fig. 1 (partial), including the brace assembly and the adjustable arm thereof.
Fig. 1e is a perspective view of the adjustable arm assembly of the apparatus of Fig. 1.
Fig. If is a perspective cutaway view of the apparatus of Fig. 1, illustrating the ratchet mechanism and associated components of the lateral positioning mechanism.
Fig. 1g is a perspective view of the brace element and adjustable arm assembly of the apparatus of Fig. 1, showing the various adjustments thereof.
Fig. 1h is a cross-sectional view of the arm assembly of Fig. 1e, taken along line 1h-1h thereof.
Fig. 1i is a perspective cutaway view of the arm assembly of Fig. 1e, taken along line 1h-1h thereof.
Fig. 1j is a perspective view of the actuator arm assembly and longitudinal element of the adjustable arm of Fig. 1e.
Fig. 2 is a perspective view of one exemplary embodiment of the alignment apparatus of the present invention, shown assembled with sensor assembly, electrical interface, and paddle.
Fig. 2a is an exploded view of the alignment apparatus of Fig. 2, showing the various components thereof.
Fig. 2b is a perspective view of the paddle device of the exemplary apparatus of Fig. 2.
Fig. 2c is a perspective view of the paddle device of Fig. 2b, with sensor assembly and electrical interface installed thereon.
Fig. 2d is a partial perspective view of the interfacing portions of paddle and first frame elements, showing the support and coupling structures associated with each.
Fig. 2e is a top plan view of a first exemplary embodiment of the electrical interface of the invention.
Fig. 2f is a top plan view of a second exemplary embodiment of the electrical interface of the invention.
Fig. 3 is a top perspective view of one exemplary embodiment of the actuator of the present invention, shown assembled.
Fig. 3a is a bottom perspective view of the actuator of Fig. 3, illustrating the coupling mechanism(s).
Fig. 3b is a cross-sectional view of the actuator of Fig. 3, illustrating the various internal components.
Fig. 3c is a side perspective view of the interior assembly of the actuator of Fig. 3, illustrating the motor and substrate assemblies thereof.
Fig. 3d is an exploded perspective view of the motor assembly of Fig. 3c.
Fig. 3e is an exploded perspective view of the sensor (applanation) drive unit used in the motor assembly of Figs. 3c and 3d.
Fig. 3f is a side cross-sectional view of an exemplary embodiment of the sensor-actuator coupling device of the invention.
Fig. 4 is a logical flow diagram illustrating one exemplary embodiment of the method of positioning a sensor according to the invention.
Fig. 5 is a logical flow diagram illustrating one exemplary embodiment of the method of performing multiple hemodynamic measurements according to the invention.
Fig. 6 is a logical block diagram of another exemplary embodiment of the system of the invention, adapted for hydrostatic correction.
Fig. 6a is graphical representation of a first exemplary screen display provided by the system of Fig. 6, showing the operation of the hydrostatic correction algorithm.
Fig. 6b is graphical representation of a second exemplary screen display provided by the system of Fig. 6, showing an optional patient orientation GUI.
Fig.7 is a logical flow diagram illustrating one exemplary embodiment of the method of providing treatment to a subject using the methods and apparatus of the present invention.

### Detailed Description of the Invention

Reference is now made to the drawings wherein like numerals refer to like parts throughout.

It is noted that while the invention is described herein primarily in terms of a method and apparatus for assessment of hemodynamic parameters of the circulatory system via the radial artery (i.e., wrist or forearm) of a human subject, the invention may also be readily embodied or adapted to monitor such parameters at other blood vessels and locations on the human body, as well as monitoring these parameters on other warm-blooded species. All such adaptations and alternate embodiments are readily implemented by those of ordinary skill in the relevant arts, and are considered to fall within the scope of the claims appended hereto.

As used herein, the term "hemodynamic parameter" is meant to include parameters associated with the circulatory system of the subject, including for example pressure (e.g., diastolic, systolic, pulse, or mean), blood flow kinetic energy, velocity, density, time-frequency distribution, the presence of stenoses, SpO₂, pulse period, as well as any artifacts relating to the pressure waveform of the subject.

Additionally, it is noted that the terms "tonometric," "tonometer," and "tonometery" as used herein are intended to broadly refer to non-invasive surface measurement of one or more hemodynamic parameters such as pressure, such as by placing a sensor in communication with the surface of the skin, although contact with the skin need not be direct (e.g., such as through a coupling medium or other interface).

The terms "applanate" and "applanation" as used herein refer to the compression (relative to a state of non-compression) of tissue, blood vessel(s), and other structures such as tendon or muscle of the subject's physiology. Similarly, an applanation "sweep" refers to one or more periods of time during which the applanation level is varied (either increasingly, decreasingly, or any combination thereof). Although generally used in the context of linear (constant velocity) position variations, the term "applanation" as used herein may conceivably take on any variety of other forms, including without limitation (i) a continuous non-linear (e.g., logarithmic) increasing or decreasing compression over time; (ii) a non-continuous or piece-wise continuous linear or non-linear compression; (iii) alternating compression and relaxation; (iv) sinusoidal or triangular waves functions; (v) random motion (such as a "random walk"; or (vi) a deterministic profile. All such forms are considered to be encompassed by the term.

### Overview

In one fundamental aspect, the present invention comprises apparatus and associated methods for accurately and repeatably (if desired) disposing one or more sensors with respect to the anatomy of a subject to facilitate subsequent hemodynamic parameter measurements using the sensor(s). For example, as will be described in greater detail below, the present invention is useful for accurately placing a pressure sensor assembly for continuously and non-invasively measuring the blood pressure from the radial artery of a human being. However, literally any kind of sensor (ultrasound, optical, etc.) can be used alone or in combination consistent with the invention, including for example the devices and associated techniques described in co-pending U.S. patent application Serial Nos. 09/815,982 entitled "Method and Apparatus for the Noninvasive Assessment of Hemodynamic Parameters Including Blood Vessel Location" filed March 22, 2001, and 09/815,080 entitled "Method and Apparatus for Assessing Hemodynamic Parameters within the Circulatory System of a Living Subject" filed March 22, 2001, both of which are assigned to the assignee.

In one exemplary embodiment, the aforementioned pressure sensor is coupled to an actuator mechanism carried by a brace assembly worn by the subject in the area of the radial artery. The actuator mechanism, when coupled to the sensor, controls the sensor lateral (and proximal, if desired) position as well as the level of applanation of the underlying tissue according to any number of control schemes, including for example that set forth in Assignee's co-pending U.S. patent application Serial No. 10/211,115 filed August 1, 2002, entitled "Method and Apparatus for Control of Non-Invasive Parameter Measurements", and in co-pending application Serial No. 10/072,508 filed February 5, 2002, entitled "Method and Apparatus for Non-Invasively Measuring Hemodynamic Parmeters Using Parametrics". However, the present invention is also compatible with systems having separate sensor(s) and applanation mechanisms, as well as combinations of the foregoing features and sensors. The actuator is advantageously "displacement" driven, and accordingly does not rely on measurements of applied force, but rather merely displacement. This approach greatly simplifies the construction and operation of the actuator (and parent control system) by obviating force sensors and signal processing relating thereto, and further makes the actuator and system more robust.

The apparatus of the present invention also advantageously maintains a highly rigid coupling between the sensor assembly and the brace element used to receive the subject's anatomy, thereby further enhancing the accuracy of the system through elimination of nearly all compliance within the apparatus.

Other significant features of the present invention include (i) ease of use under a variety of different operational environments; (ii) repeatability of measurements; and (iii) disposability of certain components. These features are achieved through the use of novel structures and techniques for placing the sensor(s) and operating the device, as well as significant modularity in design and consideration of the constraints relating to the typical (and atypical) clinical environment.

In one aspect, the present invention overcomes the disabilities associated with the prior art by providing a sensor assembly which is detachable from the parent apparatus and remains positioned on the subject during transport, thereby facilitating highly repeatable measurements using the same sensor at different physical locations within the care facility (e.g., hospital). These and other features are now described in detail.

### Apparatus for Hemodynamic Assessment

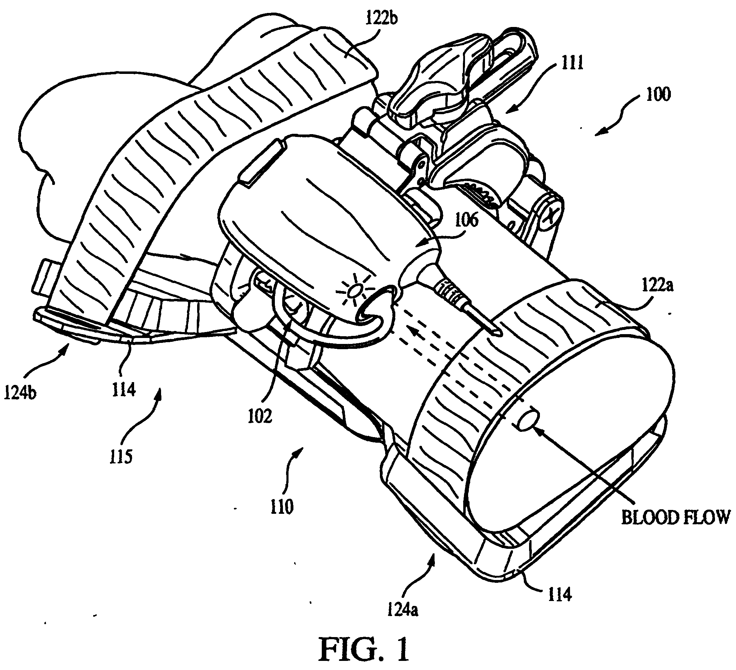

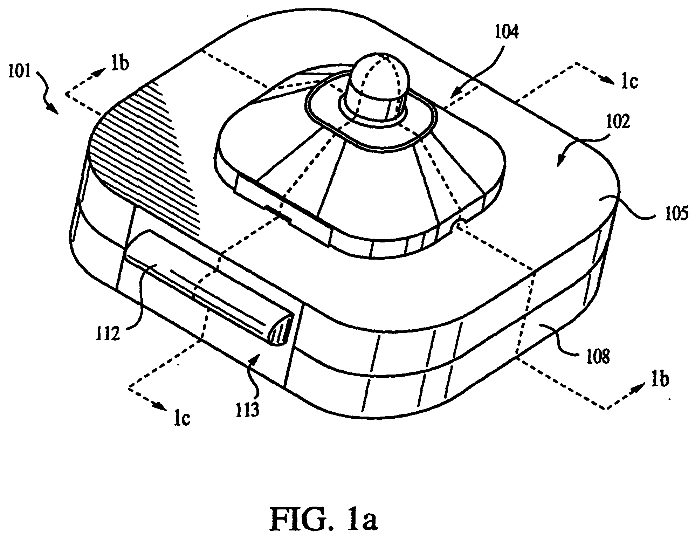

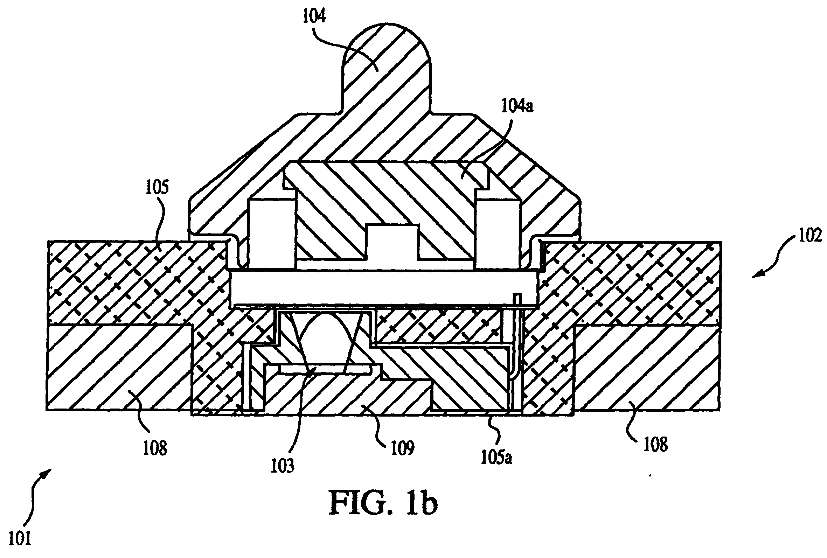

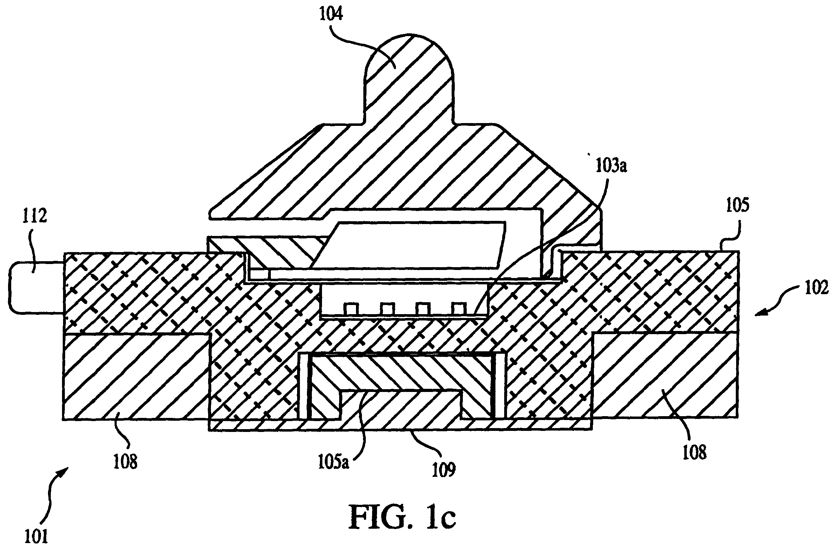

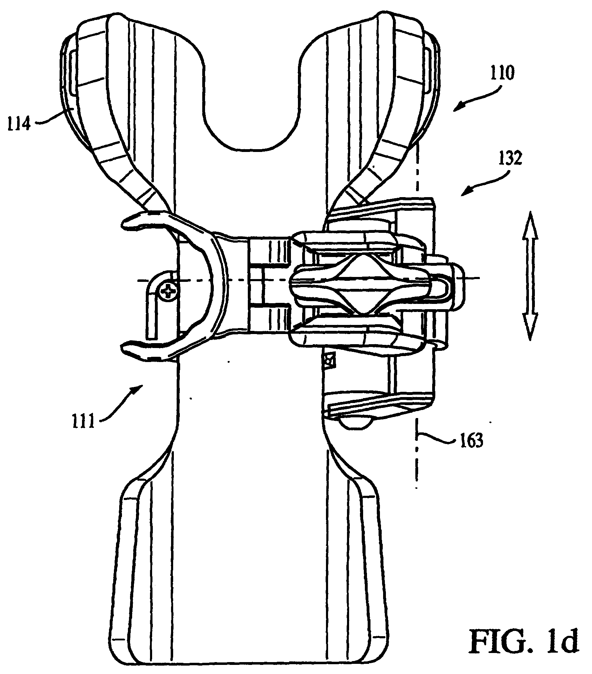

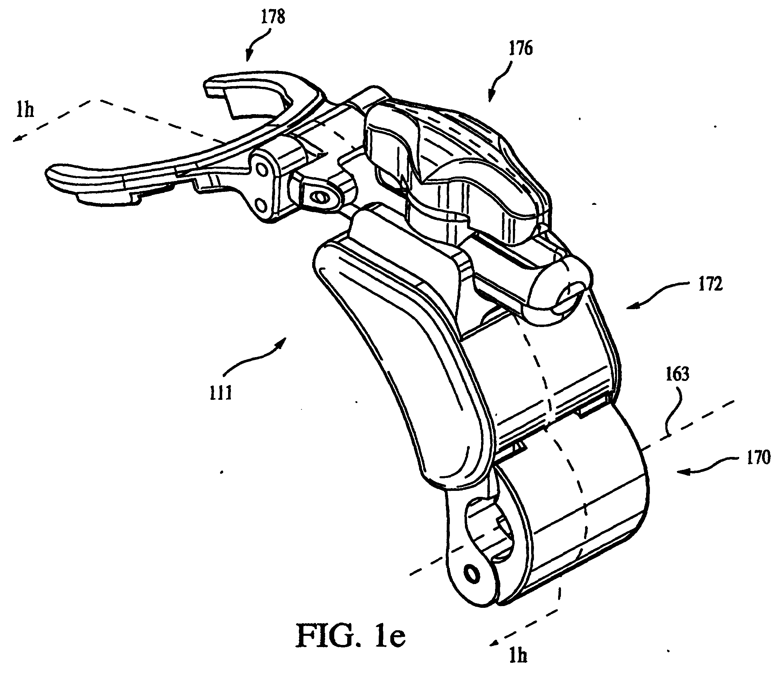

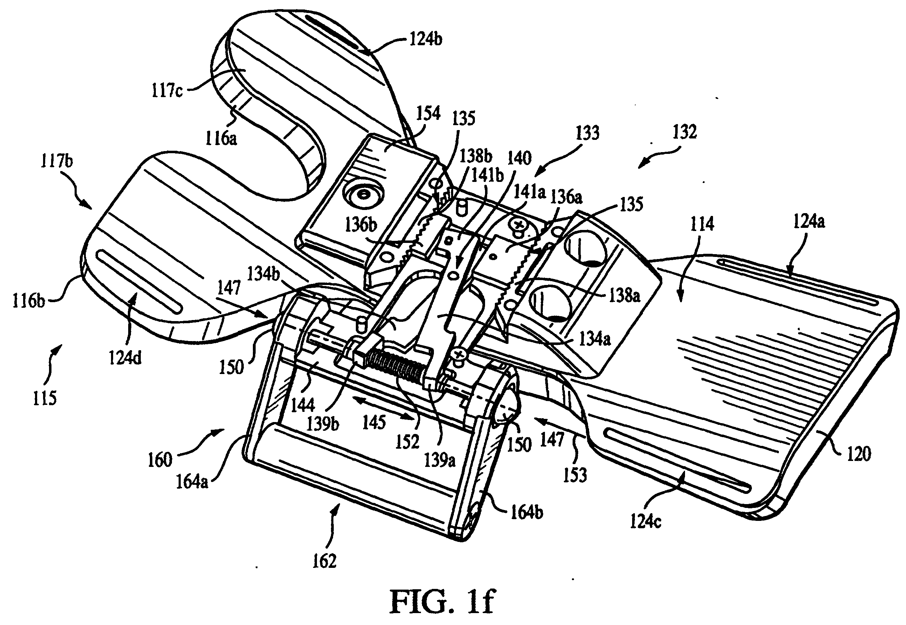

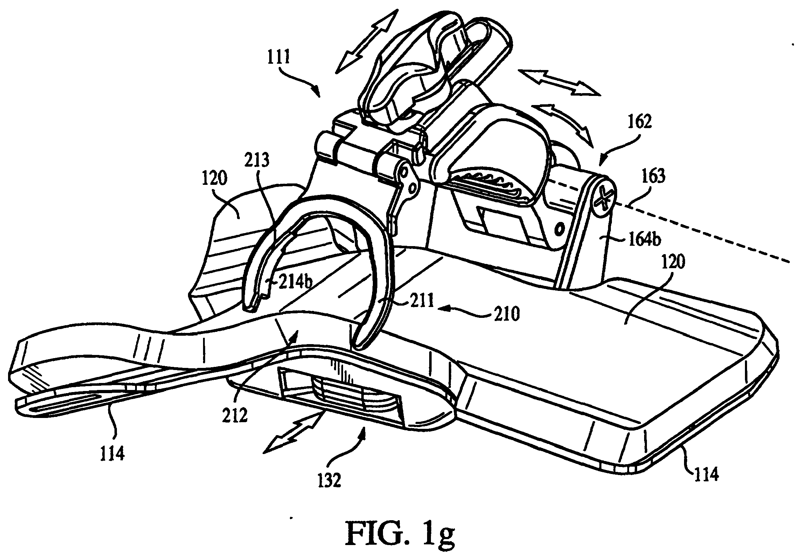

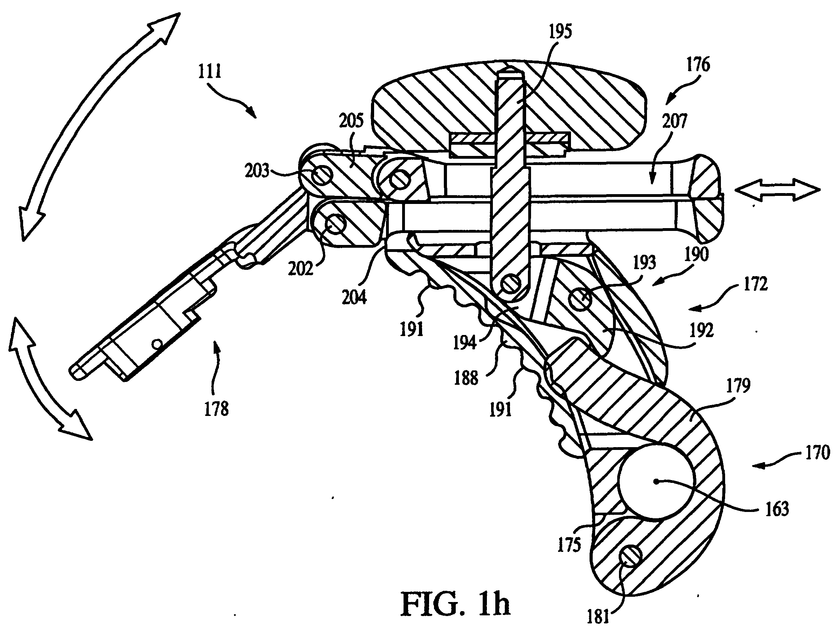

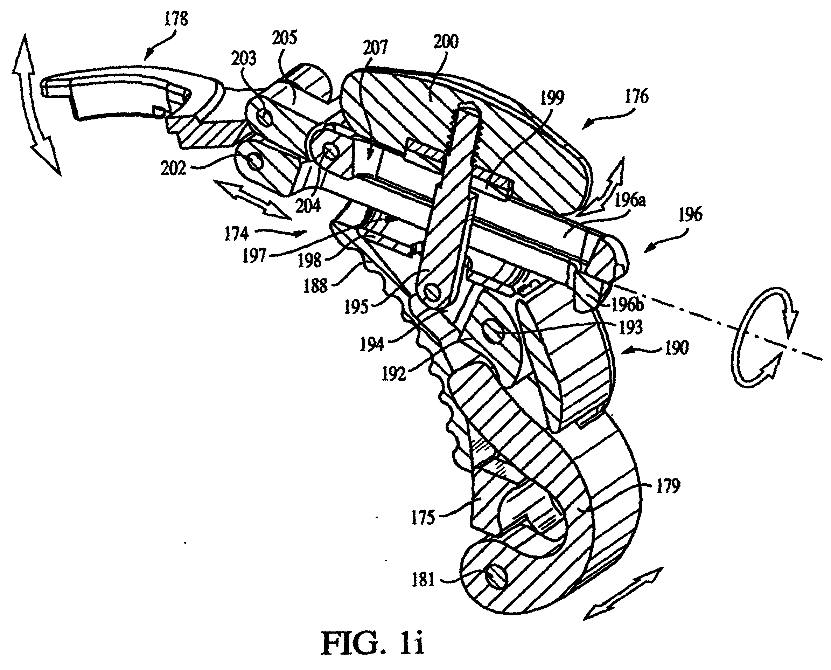

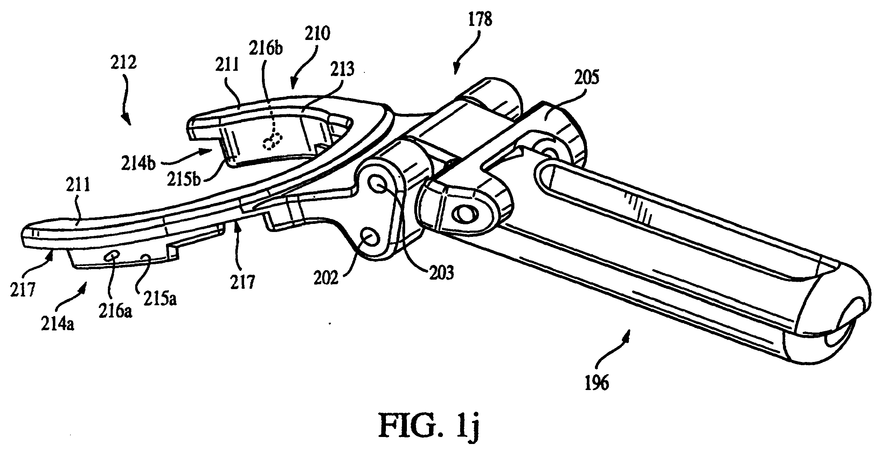

Referring now to Figs. 1- 1j, a first embodiment of the hemodynamic assessment apparatus 100 of the invention is described in detail.

It is known that the ability to accurately measure the pressure associated with a blood vessel depends largely upon the mechanical configuration of the applanation mechanism. Under the typical prior art approaches previously discussed, the pressure transducer alone comprises the applanation mechanism such that the mechanism and transducer are fixed as a single unit. Hence, the pressure transducer experiences the full force applied to deform the tissue, structures, and blood vessel. This approach neglects the component of the applantion force required to compress this interposed tissue, etc. as it relates to the pressure measured tonometrically from the blood vessel. Conversely, under no compression, the magnitude of the pressure within the blood vessel is attenuated or masked by the interposed tissue such that the pressure measured tonometrically is less than that actually existing in the vessel (so-called "transfer loss").

In contrast, the sensor assembly 101 of the present invention (see Figs. 1a -1c discussed below) embodies the pressure transducer assembly 103 disposed within an applanation element 102, the latter having a specially designed configuration adapted to mitigate the effects of such transfer loss in a simple, repeatable, and reliable way such that it can be either (i) ignored or (ii) compensated for as part of the tonometric measurement.

As shown in Fig. 1, the applanation element 102 is coupled via an actuator 106 and moveable arm assembly 111 (both described in greater detail subsequently herein) to a wrist brace assembly 110 so as to provide a platform against which the motor of the actuator 106 may exert reaction force while applanating the subject's tissue. In the illustrated embodiment, the wrist brace assembly 110 comprises a brace element 114, adapted to fit the outer wrist and hand surfaces of the subject. The brace element 114 is in the illustrated embodiment somewhat "Y" shaped when viewed in plan (Fig. 1d), with the upper portions 116a, 116b being adapted to straddle the outside surfaces of the subject's hand as best shown in Fig. 1e. The outer edges 117a, 117b of the upper portions 116 are also deflected upwards toward the subject's hand, thereby providing a cradle to positively locate the hand with respect to the brace element 114. In the illustrated embodiment, the distal end 115 of the brace element 114 is also deflected or curved out of the plane of the longitudinal portion 118 of the element 114, thereby accommodating the natural bend or contour of the human hand when slightly bent at the wrist.

In the present embodiment, the brace element 114 is advantageously formed using either a commonly available metal alloy (e.g., Aluminum 5052 H-32 alloy) or polymer (e.g., plastic), thereby allowing for low manufacturing cost, excellent ruggedness, and an insubstantial degree of compliance with the shape of the subject's tissue, although other materials such as for example a substantially inflexible polymer may be used as well. Design compliance may be built in as well if desired, for example by using a more compliant polymer for the brace element 114. Note, however, that a minimum sufficient rigidity of this component is required to accommodate the reaction forces generated by the actuator assembly 106 shown in Fig. 1. Specifically, the actuator 106 is rigidly but removably mounted to the movable arm assembly 111 shown in Fig. 1e. The brace element 114 also includes pads 120 (e.g., foam, silicone rubber, or comparable) disposed on the interior surfaces thereof to permit the use of the brace element 114 on the subject for extended periods without discomfort. These pads 120 may also be made in a composite fashion; e.g., with pads of varying thickness, material, compliance, etc. disposed in the various portions of the brace element 114.

One or more straps 122a, 122b are also fitted to the brace element 114 such that when the brace 114 is fitted to the subject's wrist and hand, the straps 122 permit the brace element 114 to be secured to the subject's arm and hand as shown in Fig. 1. In the illustrated embodiment, the straps 122 are fixedly mounted to the brace 114 at one end (such as by being sewn, snapped, or otherwise fixedly coupled through respective apertures (124a, 124b) formed in the brace element 114, the other end being free and sized to fit through respective apertures 124c, 124d formed in the opposing sides of the brace 114. In the present embodiment, the straps 122 include fasteners 123 such as Velcro patches which are disposed on the communicating faces thereof, which facilitates firmly securing the free ends of the straps 122 to the fixed ends thereof after they have been routed through their respective apertures 124c, 124d. Hence, in practice, the user or clinician simply folds the strap over the subject's arm/hand after placement thereof in the brace 114, routes the free ends through the apertures 124c, 124d, and then folds the free ends back onto their respective straps 122 such that the fasteners on each mate and secure the straps 122 and brace 114 in position.

In another exemplary embodiment (not shown), each strap 122 is secured on the back side of the brace element 114 such that the "hook" portion of the Velcor fastener is facing outward. The strap is restrained on the back side of the brace element 114 by threading the strap through both apertures 124, with one end having an over-sized element (e.g., longitudinal bar or thick tab) which will not fit through the aperture 124. The free or distal end of the strap can therefore be wrapped around the arm of the patient after insertion of the latter into the brace element 114, then back on itself such that the loop portion of the Velcro fastener (disposed on the inside surface of the distal end of the strap 122) mates comfortably with the aforementioned hook portion disposed on the back face of the brace element 114, thereby fastening the strap 122 (and brace element 114) in place around the subject's arm. This approach advantageously makes the attachment of the strap(s) 122 simple and uncomplicated, and obviates having the user thread the strap through the apertures, since the straps 122 are essentially pre-threaded at manufacture. However, this design also permits the replacement of the straps 122, such as due to damage, wear, or contamination.

The exemplary brace shown in Fig. 1 may also optionally be fitted with a hand pad (not shown) on the forward strap 122b, and the strap and hand pad routed inside the hand (i.e., between the interior of the thumb and forefinger, and across the palm). The pad is sized and shaped to fit well within the palm (grasp) of the subject. This configuration places the pad squarely in the subject's palm, such that they can wrap their fingers comfortably around the pad during measurement.

It will also be recognized that other arrangements for securing the brace to the subject's anatomy such as mechanical clasps, snaps, slings, air or fluidic bladders, adhesives, or the like may be used in place of the foregoing configuration. Literally any means of maintaining the brace element 114 in a substantially fixed position with respect to the subject's anatomy may be substituted for the configuration of Fig. 1, the latter being merely exemplary.

In another variant of the brace element 114 of the invention (not shown), adjustment for the angle of incidence of the subject's hand with respect to the wrist is provided. Specifically, it has been found by the Assignee hereof that variation of the angle of incidence of the hand with respect to the wrist can affect the accuracy of pressure measurements obtained from the radial artery. Furthermore, it has been noted that the positioning of the fingers (including the thumb) of the subject can also under certain circumstances affect the measurements obtained. While these effects are generally small in magnitude, they can have a greater significance under certain physiologic conditions and/or for certain individuals. Hence, the present invention contemplates the use of a variable geometry brace element 114 (including the distal portion 115), thereby allowing the user/caregiver to precisely set the angle of wrist incidence relative to the long bones of the forearm. This is accomplished through use of any number of different configurations, including (i) a mechanical hinge or joint (not shown) which can be adjusted to a predetermined angle, either manually by the user or automatically, such as by a motor drive, (ii) a deformable material used in the distal and wrist region of the brace element, etc. This adjustment may be kept constant across all measurements and/or subjects measured, or alternatively adjusted individually for each measurement and/or subject according to one or more criteria. Such adjustment may also be made dynamically; i.e., during one or more measurements, so as to present the system with a range of different physiologic conditions.

As one example, the adjustment may be varied until the amplitude of the maximum pulsatile pressure of the subject is achieved (as measured by a tonometric pressure sensor or other means). As another example, the pressure waveform may be measured tonometrically during a "sweep" of incidence angle of the wrist and/or fingers. In another variant, individual adjustment for the fingers and thumb relative to one another (and the brace element 114) is utilized in order to optimize pressure measurements for such individuals. Myriad different approaches for collecting data under conditions of varying wrist/finger/forearm incidence are possible consist with the invention, all such approaches being readily implemented by those of ordinary skill given the present disclosure.

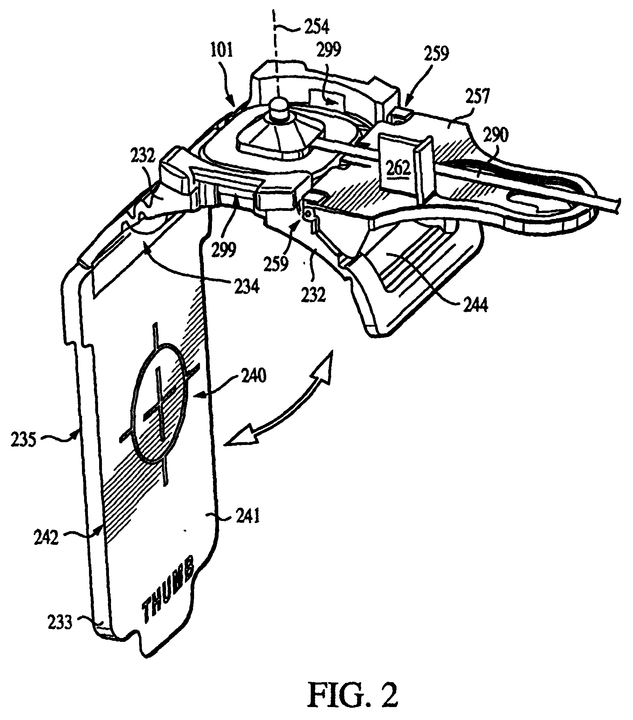

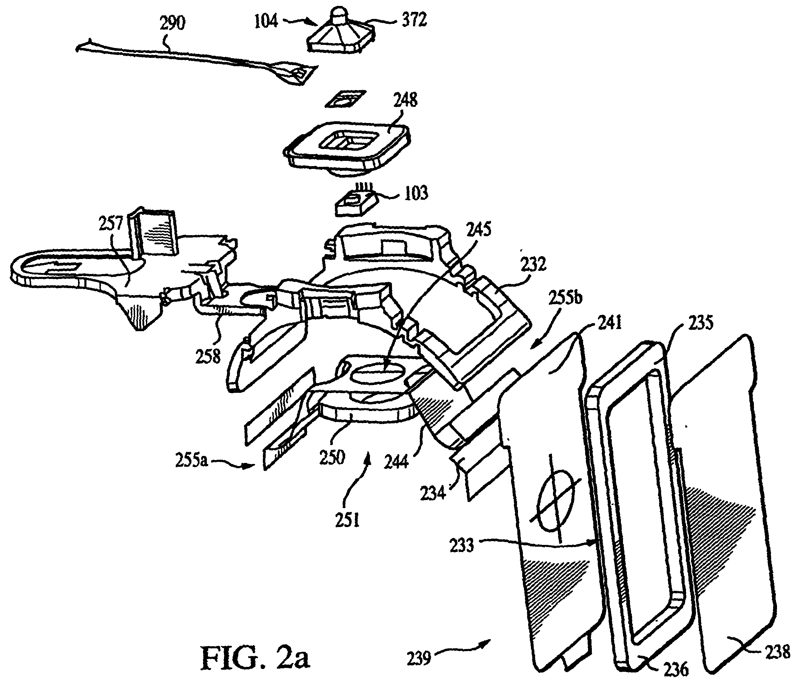

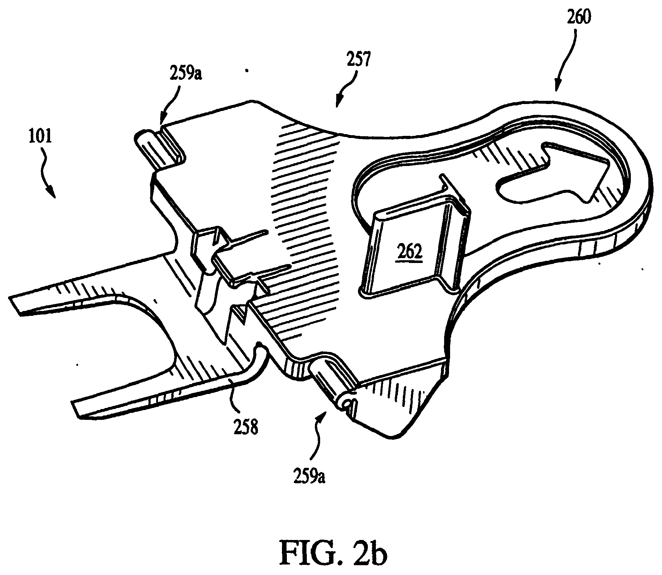

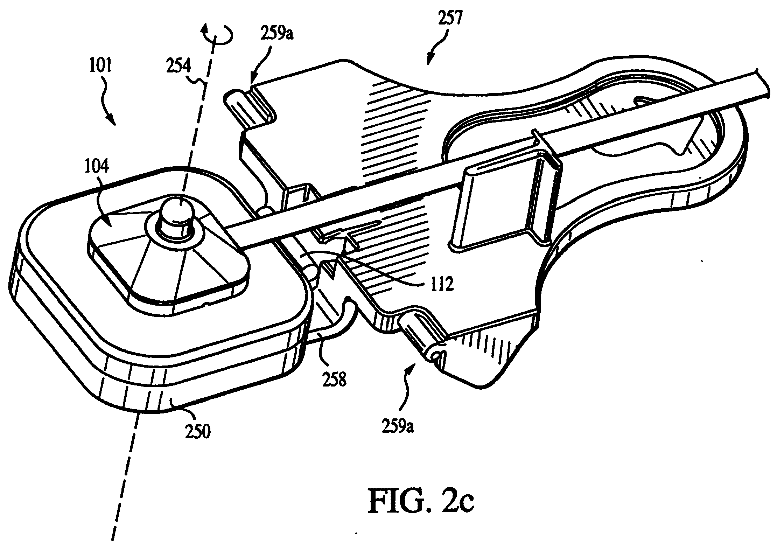

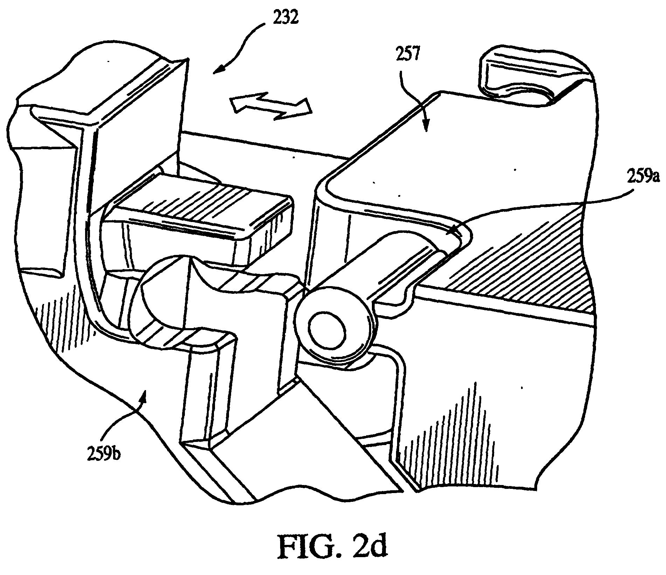

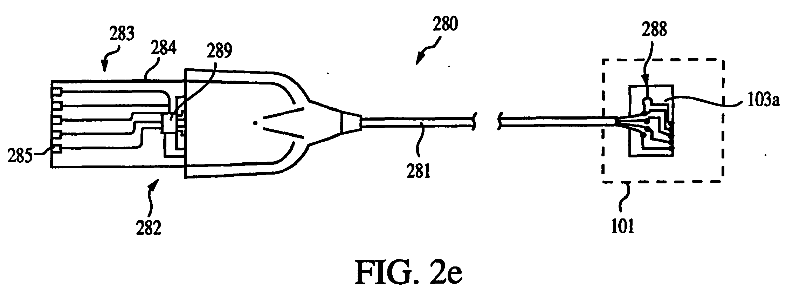

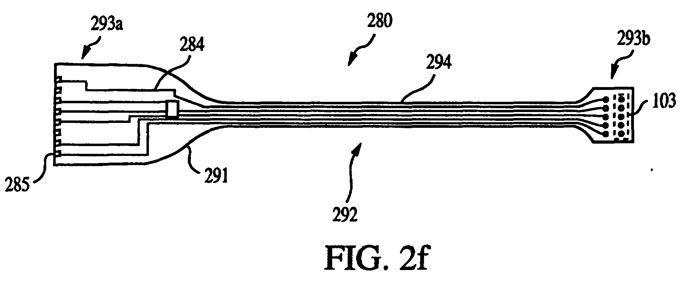

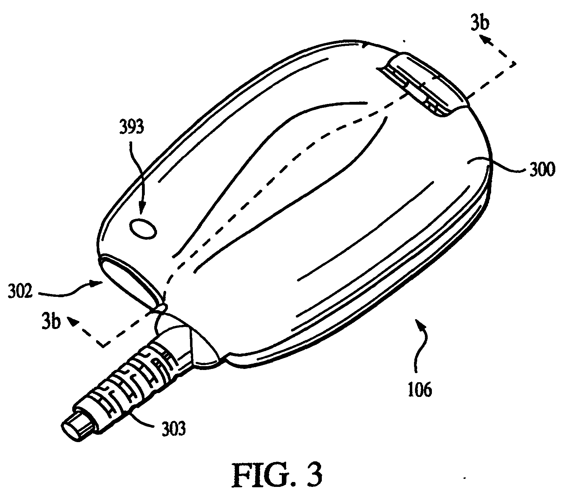

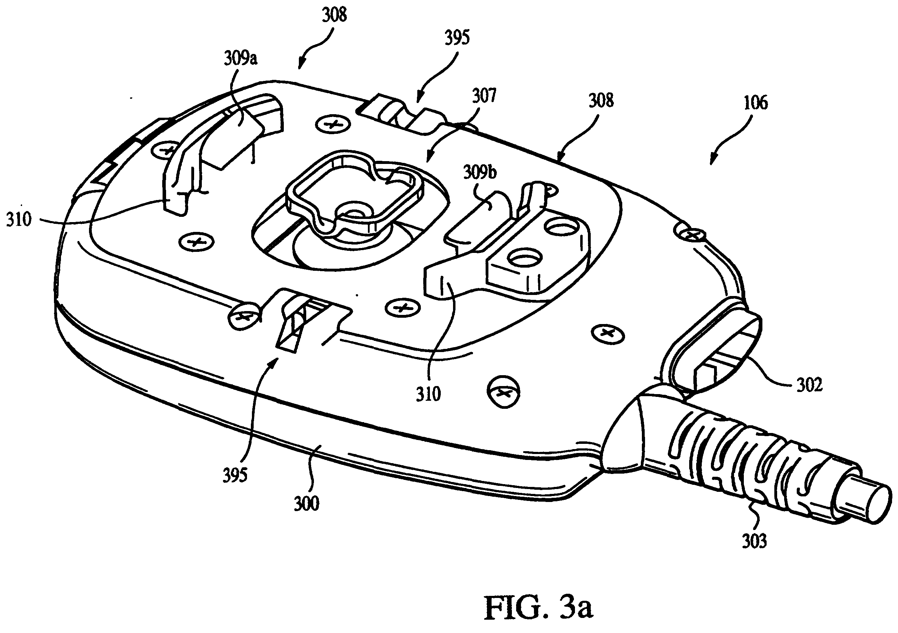

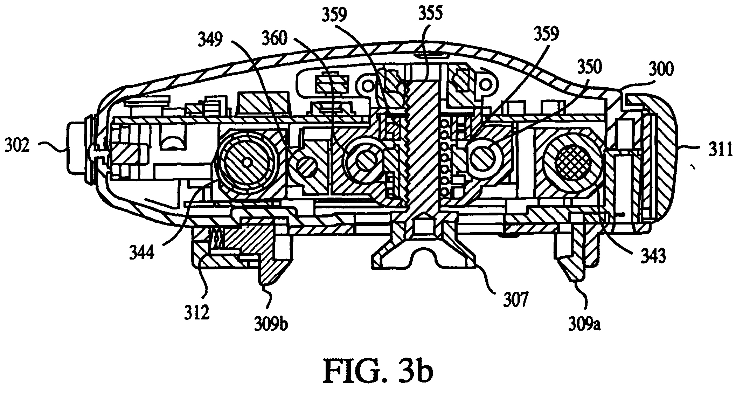

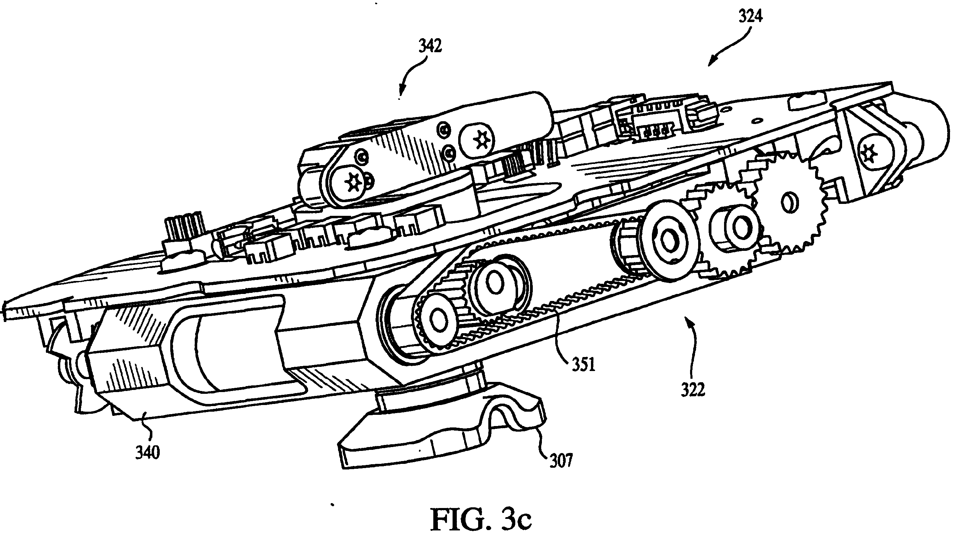

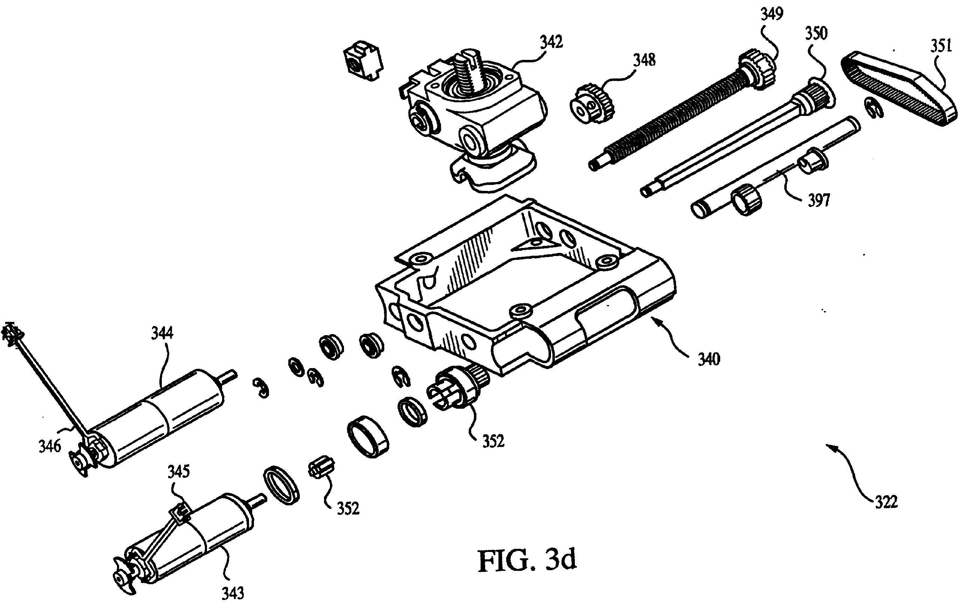

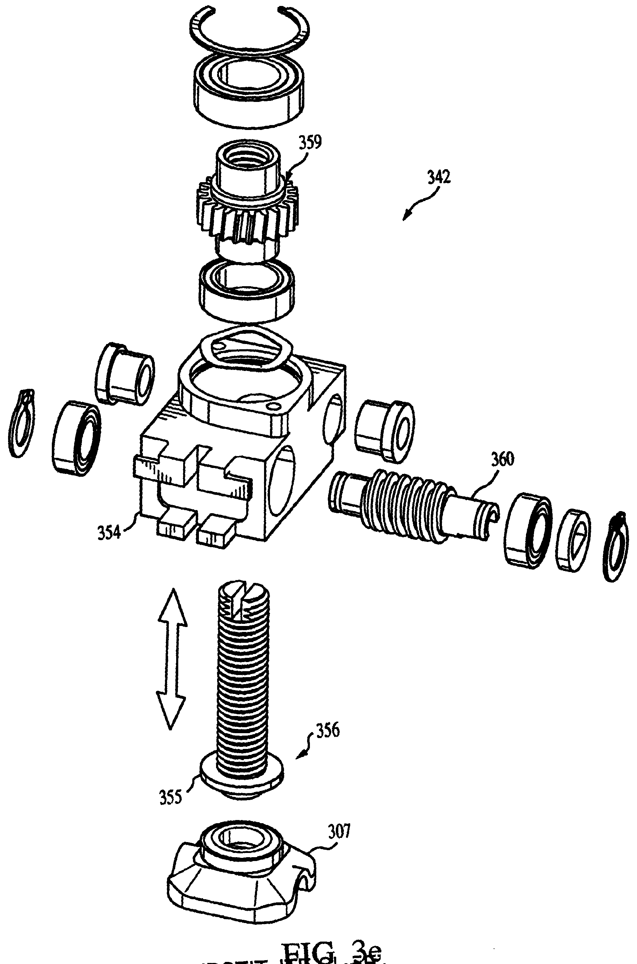

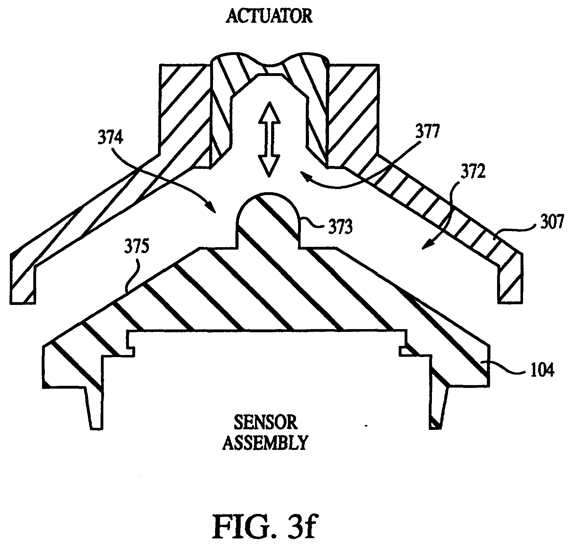

As shown in Figs. 1a-1c, the exemplary sensor assembly 101 generally comprises an applanation element 102, used to compress the tissue generally surrounding the blood vessel of interest under the force of the actuator 106, and to apply force to the blood vessel wall so as to begin to overcome the wall or hoop stress thereof. The sensor assembly 101 also includes coupling mechanism structures 104, 104a adapted to couple the sensor to its parent actuator 106 (described in greater detail below with respect to Figs. 3-3e), a housing elements 105 and 105a, pressure transducer assembly 103 with associated die 103a, strain relief device 107, and contact or bias element 108. A coupling structure 112 disposed on one face 113 of the sensor housing 105 is used to couple the sensor assembly 101 to a support structure (e.g., paddle 257, described below with respect to Figs. 2-2d) to position the sensor assembly 101 in a desired location and orientation.

It will be appreciated that while the illustrated embodiment(s) of the apparatus 100 described herein utilize the sensor assembly 101 as the applanation element, other schemes may be used consistent with the invention. For example, an actuator coupled to an applanation element (not shown) which is separate from or otherwise decoupled from the pressure or other sensor may be employed. Hence, the present invention should in no way be considered limited to embodiments wherein the sensor (assembly) also acts as the applanation mechanism. This approach does, however, simplify the associated mechanisms and signal processing considerably.

An encapsulant layer 109 comprising several mils of silicone rubber compound is applied over the active face of the pressure transducer (and selective portions of the housing 105) to provide coupling between the active face and the subject's skin, although other materials which provide sufficient pressure coupling, whether alone or used in conjunction with an external coupling medium such as a gel or liquid of the type well known in the art, may be used as well.

The bias element 108 is made from a substantially complaint foam rubber compound which acts to mitigate the effects of tissue transfer loss and other errors potentially present during tonometric measurement. Other aspects of the construction and operation of applanation element 102 are described in aforementioned U.S. patent application Serial No. 10/072,508.

It will also be recognized that the sensor and applanation element configuration of Figs. 1a-1c is merely exemplary, and other sensor configurations (e. g., single or multiple transducer, alone or combined with other types of sensors, and/or using different bias element geometry) may be used consistent with the present invention.

Referring now to Figs. 1d, 1e, and If, one exemplary embodiment of the moveable arm assembly 111 and supporting structure is described in detail. As shown in Fig. 1d, the brace element 114 includes a lateral positioning mechanism 132 which permits the moveable arm 111 (and its associated support structure, described below) to move relative to the brace element 114. In the illustrated embodiment, the lateral positioning mechanism 132 comprises a ratchet mechanism 133 (Fig. If) which is controlled by the clinician or operator to adjust the arm assembly 111 to the proper position. As shown in Fig. If, the ratchet mechanism 133 comprises two transverse ratchet arms 134a, 134b each communicating with dogs 136a, 136b having toothed engagement regions 135 disposed thereon, the toothed regions 135 adapted to engage corresponding toothed regions of respective guide members 138a, 138b. The ratchet arms 134 are both pivoted at a central pivot point 140, such that outward forces 145 applied to the arms 134 at their distal ends 139a, 139b pivot the engagement portions 141 of the arms 134, driving respective ones of the dogs 136 into engagement with the guide members 138. The dogs 136 are adapted to slide outward (i.e., longitudinally along the length of the brace 114) into toothed engagement with the toothed regions of the guide members 138, thereby locking the arms 134 (and the underlying frame element 144 to which the arms 134 are attached) in position with respect to the fixed guide elements 138.

Conversely, when inward forces 147 are applied to the distal ends of the arms 134 (such as via the adjustment buttons 150 shown in Fig. If), the engagement portions 141 of the arms 134 are retracted away from the guide members, thereby retracting the dogs 136 and allowing the frame element 144 to slide laterally (i.e., transversely across the brace element 114) until the buttons 150 are released, at which point spring tension created via one or more spring(s) 152 disposed longitudinally along the axis 153 of the buttons 150 causes the distal ends of the arms 134 to move outward, thereby re-engaging the dogs 136 with the guide members 138. The ratchet assembly 132 is further optionally outfitted with stop elements 155 which limit the outward travel of the frame element 144 and other associated components; however, in the illustrated embodiment, such stop elements are not utilized so as to allow the frame element 144 and associated components to be removed and swapped (inverted) with respect to the brace element 114. Specifically, the brace element 114 (and lateral positioning mechanism) are designed to be symmetrically applied to the subject, such that the brace element can be applied to either arm of the subject.

The design of the ratchet mechanism 132 of Fig. 1f also advantageously provides a low vertical (sagittal) profile, thereby minimizing the installed height and general bulkiness of the apparatus 100 as a whole. Furthermore, the bottom surface 154 is in the present embodiment made flat; hence, the brace 114 with mechanism 132 can be readily rested upon most any surface without imparting instability to the apparatus (or having the subject feel that their arms is precariously poised). It will further be appreciated that the bottom face 154 of the ratchet mechanism 132 can be adapted to couple with fixed or movable assemblies (not shown), which may keep the apparatus in a desirable orientation or location. For example, permanent magnets or ferrous elements may be disposed in the bottom face 154 or there about to allow magnetic coupling of the brace to a corresponding fixed assembly via a magnetic field, such as where it desirable to maintain the arm of a patient absolutely steady during surgery. Alternatively, a ball-and-socket arrangement may be used wherein the brace element 114 can rotate in multiple degrees of freedom around the ball thereby allowing the subject's arm to move, yet with restriction in the lateral, proximal, and normal directions. Myriad other approaches for controlling the position of the brace element (whether while in use or otherwise) may be utilized consistent with the present invention, all such approaches being readily implemented by those of ordinary skill in the relevant art.

As shown in Fig. If, the ratchet mechanism 132 further comprises a coupling frame 160 which is fixedly mounted to the frame element 144 of the mechanism 132. The coupling frame 160 comprises in the illustrated embodiment a transverse bar 162 which is disposed in longitudinal (i.e., proximal) orientation between two frame arms 164a, 164. The transverse bar 162, as best shown in Fig. 1g, allows for the support of the moveable arm 111 and the rotational adjustment thereof (i.e., rotation of the arm 111 around the axis 163 of the bar 162), as well as longitudinal (proximal) adjustment of the arm 111 along the length of the bar 162. Hence, when the frame element 144 of the ratchet 132 slides laterally in and out of the brace 114, the coupling frame 160 and its transverse bar 162 move accordingly.

The moving arm assembly 111 is now described in detail. As shown best in Fig. 1e, the moving arm assembly 111 comprises four primary sections or components, including (i) a coupling element 170 adapted for mating with the transverse bar 162 of the coupling frame 160; (ii) a support section 172 joined to the coupling element 170; (iii) a lateral adjustment mechanism 176 disposed at the distal end 174 of the support section 172; and (iv) an actuator arm 178 coupled to the lateral adjustment mechanism 176. Collectively, and when considered in conjunction with the ratchet mechanism 132 previously described with respect to Fig. If, these components allow for the adjustment of the actuator arm 178 (and hence actuator 106 and sensor assembly 101) over several degrees of freedom. As will be described in greater detail herein, this feature advantageously allows the user or caregiver to position the sensor assembly 101 in literally any orientation with respect to the surface of the subject's skin, yet also tends to properly align the actuator and sensor element for the user/caregiver, thereby simplifying operation of the apparatus and system as a whole. As described below, the moveable arm apparatus 111 of the present embodiment also includes design features whereby multiple degrees of freedom are secured/released by the user during the adjustment process, thereby even further simplifying the adjustment and use of the device.

Referring to Fig. 1h, the coupling element 170 of the movable arm 111 comprises a block element 175 which cooperates with a moveable lever element 179 to rigidly yet adjustably grasp the transverse bar 162. Specifically, the block element is pivotally mated to the lever 179 via a pivot pin 181, such that the two components may rotate around the pivot 181 with respect to each other. The block element 175 is captured within the curved body section 190 of the support section 172 (described below), such that the position of the lever 179 controls the relative friction applied between the two components 175, 179 and the surface of the transverse bar 162. As will be set forth in greater detail subsequently herein, the position of the lever 179 is controlled through the action of the operator when adjusting the lateral position of the actuator arm 178 via the lateral position mechanism 176. It will be appreciated that while a smooth surface is used for the transverse bar 162 and interior mating faces of the block element 175 and lever, any number of other surface finishes and/or configurations may be used to facilitate greater or lesser frictional capability, including for example uneven or rough textures, or even toothed splines.

The support section 172 of the illustrated embodiment comprises a substantially rigid, curved body frame 190 adapted to generally match the contour of the subject's forearm. The body section in the exemplary embodiment is fabricated from 6061 T-6 aluminum alloy, although it will be recognized that the part(s) could be made from a casting alloy, molded plastic, or even composite material (if designed to accommodate the stresses in the part.) The use of the T-6 aluminum alloy provides light weight yet good rigidity and other mechanical properties. The interior surface 192 of the support section 172 includes a foam, elastomeric (e.g., silicone) rubber, or soft urethane pad 188 adapted to firmly but gently mate with the subject's skin when the arm assembly 111 is locked in place, such that relative movement between the support section 172 and subject's skin is minimized. Reduction of relative movement is accomplished primarily via friction which is enhanced through the use of a plurality of surface features 191 of the pad 188 (e.g., serrations in the present embodiment, although other features such as hemispherical bumps, or alternatively other approaches such as surface adhesion may be utilized). This reduction in relative movement helps stabilize the apparatus 100 as a whole and avoid relative movement of the sensor assembly 100 and the subject's anatomy, thereby permitting more accurate and repeatable measurements. The serrations or grooves also help ensure peripheral blood flow even if the pad is improperly applied (e.g., made excessively tight against the skin of the subject).

As previously described, the support section 172 contains at least partly the blocking element 175 and lever 179 which cooperate to adjustably capture the transverse bar 162. In the illustrated embodiment, the body frame 190 of the support section 172 acts as a frame which provides support for the various other components, including the lever 179 and blocking element 175. Specifically, the blocking element 175 is rigidly mated to the body frame 190 (such as via welding, riveting, threaded fastener, or even forming the two components as one during fabrication). A second lever 192 pivoted around a pivot point 193 supported by the body frame 190 engages the first lever 179 at a distal point of the latter, thereby controlling the amount of frictional force applied by the mating surfaces of the first lever 179 to the transverse bar 162. In the illustrated embodiment, the opposing end 194 of the second lever 192 is coupled (via pivot) to the threaded shaft 195 of the lateral adjustment mechanism 176 (described below), thereby allowing the user to control multiple degrees of freedom of the moveable arm 111 simultaneously; i.e., the adjustment of the lateral positioning mechanism 176, and the degree of rotation of the coupling element 170 and support section 172 around the transverse bar 162. The support section 172 and coupling element 170 collectively rotate around the axis 163 of the transverse bar 162 of the coupling frame 160, thereby allowing adjustment of the apparatus to fit different individuals, and further permitting un-obscured access of the arm to the brace element 114 during installation of the apparatus 100 on the subject.

As shown best in Figs. 1h and 1i, the distal portion 174 of the body section is also adapted to receive the lateral adjustment mechanism 176, the latter being used in conjunction with the ratchet mechanism 132 previously described to adjust the "coarse" lateral (i.e., transverse) position of the sensor assembly 101 and actuator 106 prior to operation. As used herein, the terms "coarse" and "fine" are relative, the former generally referring to the process of positioning the moveable arm assembly 111 during installation of the apparatus 100 on the subject being monitored, while the latter generally refers to the smaller-scale positional adjustments conducted by the actuator assembly 106 during operation (described in detail below). Specifically, in the present embodiment, the user may, after fitting the brace element 114 and straps 122 to the subject's arm, adjust the ratchet mechanism 132 (by depressing the buttons 150 on the sides thereof as previously described) and sliding the frame element 144 laterally in or out as appropriate, thereby affecting the position of the moveable arm 111 including the actuator arm 178. Thereafter, the user may then utilize the lateral adjustment mechanism 176 of the moveable arm assembly 111 to further adjust the position of the actuator arm 178 as desired.

The adjustment mechanism 176 comprises, in the illustrated embodiment, a split-pin arrangement wherein a central longitudinal element 196 comprising first and second portions 196a, 196b is disposed within a corresponding channel 197 formed between a lower guide element 198 and an upper guide element 199. The mechanism 176 further includes an adjustment knob 200 which is threadedly engaged with the threaded fastener 195 previously described. As one turns the knob 200 in the counterclockwise (CCW) direction, the fastener 195 is progressively disengaged, thereby reducing the rotational force on the second lever 192, which in turn reduces the frictional force on the transverse bar 162. Concurrently, the frictional force on the split longitudinal element 196 is reduced, thereby allowing movement of the first and second portions thereof 196a, 196b relative to one another (and the upper and lower guide elements 199, 198).

As best shown in Figs. 1h and 1i, the aforementioned relative movement of the first and second portions 196a, 196b imparts an additional degree of freedom to the actuator arm 178. Specifically, the actuator arm of the illustrated embodiment employs a three-pivot arrangement wherein first, second and third pivots 202 and 203, and 204 are coupled to the first and second portions 196a, 196b respectively (and an intermediary linlc 205), such that when the first and second portions 196a, 196b slide longitudinally in relation to one another, the relative positions of the first and third pivots 202, 204 change, thereby altering the angular displacement 206 of the actuator arm 178.

The longitudinal element 196 further includes an aperture 207 formed vertically along at least a portion of the length of the element 196, thereby permitting the threaded fastener 195 to penetrate there through. This feature advantageously makes the assembly self-limiting; i.e., the shaft of the threaded fastener 195 acts to capture the longitudinal element 196 at the limit(s) of its travel. This configuration further helps to maintain a desired degree of rotational alignment of the actuator arm 178 with respect to the rest of the movable arm assembly 111. In the illustrated embodiment, the aperture 207 and longitudinal element 196 cooperate to allow a limited degree of rotation of the element 196 (and hence the actuator arm 178), thereby accommodating adjustment of the arm 178 so as to match the orientation of the sensor frame to the other components of the apparatus 100.

In the illustrated embodiment, the aperture 207 has ten-degree (10°) sides machined into the longitudinal element 196 to allow for such rotation.

Hence, by rotating one knob 200, the user can readily free or alternatively "freeze" multiple degrees of freedom within the movable arm assembly 111, namely (i) the rotation of the moveable arm assembly 111 around the transverse bar 162; (ii) the proximal-distal movement of the arm assembly 111 on the transverse bar 162 (iii) the lateral position of the central longitudinal element 196 within its guide channel 197; (iv) the angular displacement of the actuator arm assembly 178 relative to the support element 172 (via relative movement of the first and second portions 196a, 196b); and (v) the "limited" angular rotation of the longitudinal element 196 in its guide channel 197 via the slot 207. Additionally, it will be recognized that while a fastener 195 and aperture 207 formed in each of the first and second portions 196a, 196b are used to cooperatively control both the limit of transverse travel and rotation of the actuator arm 178 and longitudinal element 196, other arrangements which do not so limit these parameters may be used. For example, if desired, the apparatus 111 may be configured such that the rotation of the longitudinal member 196 is controlled independently of the threaded fastener 195, such as by offsetting the axis of the member 196 from the fastener 195, and controlling the friction applied thereto by a transverse plate or structure.

Referring now to Figs. 1g and 1j, the distal portion 210 of the actuator arm 178 is described in detail. As previously discussed, the actuator arm 178 is adapted to receive the actuator assembly 106 during normal operation, thereby providing the actuator with, *inter alia,* a reaction force (i.e., a structure against which to exert applanation force on the subject's blood vessel). As described in greater detail below, the distal portion 210 of the actuator arm 178 also interfaces with an alignment apparatus (Fig. 2 below) to position and maintain the sensor (e.g., the sensor assembly 101 of Fig. 1) with respect to the blood vessel, especially (i) prior to first attachment of the actuator 106 to the assembly 100; and (ii) after the actuator has been attached, and then subsequently removed from the assembly 100, such as during transfer of the subject from the operating room to a recovery room. As shown in Figs. 1g and 1j, the distal portion 210 includes a horseshoe or "U" shaped arm portion 211 with an opening 212 disposed on the side opposite the coupling of the arm 178 to the longitudinal element 196. The arm 178 including the distal portion 210 are made substantially rigid in the illustrated embodiment (i.e., fabricated out of a lightweight alloy), thereby mitigating compliance during positioning and mating with the aforementioned alignment apparatus. It will be recognized that while a U-shaped arm portion is utilized in the present embodiment, other shapes (with opening 212 or otherwise) may be substituted with equal success. The distal portion 210 further includes two skirt portions 214a, 214b which are disposed on the underside (i.e., sensor side) of the U-shaped arm portion 211 at the inner radius 213 thereof, and which act to further guide and engage the sensor assembly 101 when the latter is mated to the arm 178. Specifically, in one embodiment, the outer surfaces 215a, 215b of the skirts 214a, 214b each have a respective raised pin or dowel 216a, 216b disposed in the radial direction diametrically opposite one another, which engage with corresponding apertures 299 formed in corresponding inner surfaces of the aforementioned alignment assembly. This arrangement, *inter alia,* allows some degree of relative movement between the components, and some degree of radial misalignment ("yaw") between the actuator arm 178 and the alignment apparatus 230, as described in greater detail below. Disposing the skirt portions 214 at the inner radius 213 further provides a lip 217 around at least portions of the U-shaped arm 211, thereby providing a bearing surface 218 (i.e., the underside of the lip 217) which absorbs some of the reaction force from the alignment assembly when the two are mated, and provides a more positive and stable engagement there between.

It is noted that the apparatus 100 of the present invention is advantageously configured to maintain a highly rigid relationship between the various components, including the brace element 114, U-shaped arm 211, movable arm 111 and sensor assembly 101. Specifically, the components are designed for very limited compliance such that reaction forces generated by the act of pressing the sensor assembly 101 against the subject's tissue are in effect completely transferred via the actuator 106, arm 111, and ratchet mechanism 132 to the brace element 114, and accordingly to the tissue on the back side of the subject's forearm. This high degree of rigidity allows for increased accuracy in the tonometric pressure measurement, since variations in the measured pressure resulting from the compliance of various portions of the apparatus are virtually eliminated.

Similarly, the pads 120, 188 of the exemplary apparatus are designed with a comparatively large surface or contact area to the subject's tissue, such that the reaction forces transmitted via the apparatus 100 to the pads are distributed across a large are of tissue, thereby further mitigating the effects of compliance.

Referring now to Figs. 2 through 2d, one exemplary embodiment of the alignment apparatus 230 (and associated components) is described in detail. It will be recognized that while termed an "alignment apparatus" in the present description, the apparatus of Figs. 2-2d has several functions, including (i) general alignment of the actuator 106 and the sensor assembly 101 within the apparatus 230 so as to facilitate coupling of the two components; (ii) support of the paddle 257 (described below) which maintains the sensor in an initial orientation during actuator coupling and sensor calibration; and (iii) retention of the sensor assembly 101 within the apparatus 230 after the actuator (and paddle 257) have been removed ("tethering").

As shown in Figs. 2 and 2a, the alignment apparatus in one fundamental aspect generally comprises a structure which positions the sensor assembly 101. In the illustrated embodiment, this structure is made disposable through use of inexpensive materials and design features facilitating such disposability. The apparatus 230 generally comprises a first frame element 232 and second frame element 233, which are coupled to each other via a coupling 234 such that the two frame elements 232, 233 can move relative to one another. The illustrated coupling 234 comprises a flexible polymer sheet "hinge" of the type well known in the art, although it will be appreciated that myriad other arrangements may be used, including for example an actual pin-based hinge, a fabric hinge, one or more tethers, or alternatively no coupling at all.

The first frame element 232 is in the illustrated embodiment a substantially rigid (albeit somewhat compliant) polymer molding formed from polyethylene, although other materials and degrees of flexibility may be used. The Assignee hereof has found that the medial portion of the wrist of most humans is substantially similar and has similar curvature, therefore lending itself to use of a frame element 232 which can be applied to most any person. The aforementioned level of flexibility is selected to permit some deformation of and accommodation by the frame element 232 to the shape and radius of the wrist of the subject (and cooperation with the second frame element 233, described in greater detail below). This arrangement advantageously allows for a "one size fits all" frame element 232, thereby obviating any selection process associated with a more rigid frame, and simplifying the use of the apparatus 230 overall. However, an adjustable or selectively compliant frame element may also be utilized if desired.

As will be described in greater detail below, the first frame element 232 also captures the sensor assembly 101, thereby maintaining the two components 232, 101 in a loosely coupled but substantially fixed relationship.

The second frame element 233 is made of substantially flexible polymer; i.e., polyethylene foam, although other materials and levels of flexibility up to and including inflexible materials may be used if desired. The second frame element 233 is adapted to mate with the first element 232, and further includes an adhesive 235 on its underside 236 such that when the element 233 is disposed atop the subject's skin, it bonds to the skin, the frame element 233 deforming somewhat to match the surface contour of the skin. The adhesive is advantageously selected so as to provide a firm and long-lasting bond, yet be readily removed when disposal is desired without significant discomfort to the subject; however, other means for maintaining the second frame element 233 in a constant position with respect to the subject's anatomy may be used, including for example Velcro straps, tape, etc.

A low-cost removable backing sheet 238 (e.g., waxed or coated on one side) of the type well known in the adhesive arts is used to cover the adhesive 235 prior to use to preclude compromise thereof. The user simply peels off the backing sheet 238, places the frame element 233, and gently compresses it against the subject's skin to form the aforementioned bond, deforming the second frame element as needed to the contour of the subject's anatomy. The coupling 234 allows the user/operator to simply fold the first frame element 232 over onto the top of the second element 233 after the attachment of the latter to the subject as previously described, such that the first frame element 232 straddles and sits atop the second element 233 to form a substantially unitary assembly when adhesively bonded.

The second frame element 233 of the illustrated embodiment further includes an alignment device 239 which aids the user/operator in properly positioning the second frame element 233 at the onset. In the illustrated embodiment, this alignment device comprises a reticle 240 disposed upon a substantially transparent and removable alignment sheet of polymer 241 (e.g., clear polyester or polyethylene) which is also removably affixed to the second frame 233 on its top surface 242 via an adhesive. Hence, once the desired specific monitoring location has been identified (such as by the user/operator finding a suitable pulse point on the surface of the subject's medial region using their finger or other technique), the backing sheet 238 is peeled off, and the reticle 240 of the second frame 233 aligned over the pulse point. The user/operator then simply presses the adhesive surface 235 against the subject's skin to affix the second frame in place, and subsequently peels off the alignment sheet 241. Peeling off the alignment sheet 241 from the top surface of the second frame 233 in the illustrated embodiment exposes additional adhesive, which is used to bond the first frame element 232 to the second 233 when the two are ultimately mated. Hence, the adhesive on the top portion of the second element 233 serves two functions: (i) to initially maintain the alignment sheet 241 in place; and (ii) to maintain a fixed relationship between the first and second frame elements 232, 233 when the two are mated.

It will be recognized, however, that other arrangements for coupling the first and second frame elements 232, 233 may be utilized in place of the adhesives of the present embodiment. For example, a mechanical linkage (e.g., clasp, clip, or frictional pin) arrangement may be used. Alternatively, the two frames could be provided as a unitary element (not shown) with adhesive on its bottom (tissue) side, wherein the alignment sheet 241 with reticle is extracted laterally via a guide slot formed within the unitary frame after placement of the frame. As yet another alternative, a partial frame (i.e., only covering a portion of the subject's medial area) could be employed. Yet even other variants of the basic concept of the alignment apparatus; i.e., a structure having an associated alignment mechanism for accurately disposing one or more sensors over the pulse point, will be recognized by those of ordinary skill in the mechanical arts, and accordingly are not described further herein.

Since the coupling relationship between the first and second frame elements 232, 233 is in the illustrated embodiment substantially fixed, the first frame 232 is then folded atop the second 233, thereby aligning the first frame 232 with respect to the pulse point (i.e., the pulse point is now disposed in a substantially central position within the boundaries of the first and second frames 232, 234). This is significant from the standpoint that the sensor assembly 101, by virtue of its indirect coupling to the first frame element 232, is now also at least coarsely aligned with the pulse point on the subject's wrist. From this point forward, and even during multiple subsequent measurements wherein the brace 100 and actuator 106 are removed and repositioned, the user/operator need not again reposition the sensor, a distinct benefit in environments where such multiple measurements are conducted.

As shown best in Figs. 2 and 2b, the sensor assembly 101 of the present embodiment is coupled to the first frame 232 using a selectively lockable suspension arrangement; i.e., the sensor assembly 101 is loosely coupled and suspended within the frame 232 via the actuator 106 when unloclced, and rigidly coupled in the frame 232 when locked. Suspension of the sensor assembly 101 (i.e., the unloclced state) is desirable during use, when the actuator 106 is coupled to the sensor assembly 101, and is controlling its movement. The locked state is desirable, *inter alia,* when initially positioning the sensor (and parent alignment apparatus 230) on the subject, and when coupling the actuator 106 to the sensor assembly 101.

Coupling of the sensor assembly 101 to the frame element 232 is accomplished using a flexible suspension sheet 244 which is coupled rigidly to the first frame 232 such as via adhesive or other means. The suspension sheet 244 includes an aperture 245 in its central region, through which the sensor assembly 101 mates. Specifically, the pressure transducer 103 and associated portions of the housing 105 protrude through the aperture 245 such that they are below the plane of the sheet 244 in that region. The contact pad 108 is disposed on the tissue (contact) side 251 of the sheet 244, and mated by adhesive (e.g., acrylic adhesive of the type well known in the art) to the sheet 244 and the exposed portions of the bottom face of the housing 105, thereby forming an assembly which has the sheet 244 securely captured between the contact pad 108 and the housing 105, with the sensor (e.g., pressure transducer) protruding through both the aperture 245 in the sheet 244 and the aperture 252 formed in the contact pad 108.

The suspension sheet 244 is in the present embodiment provided sufficient extra surface area and "slack" such that when the sheet 244 is captured by its ends 255a, 255b within the first frame element 232, the sensor assembly 101 can move to an appreciable degree laterally within the frame 232, thereby allowing the actuator 106 to move the sensor assembly 101 laterally across the radial artery during its positioning algorithm. The present invention also contemplates such freedom of movement in the proximal direction as well. For example, sufficient play may be provided in the suspension sheet 244 to allow a small degree of proximal movement of the sensor assembly 101 by the actuator 106. Furthermore, when using an elastomer or other highly compliant material, rotation of the sensor assembly 101 in the X-Y plane (i.e., "yaw" of the sensor assembly about its vertical axis 254) can be accommodated. Other arrangements may also be used, such alternatives being readily implemented by those of ordinary skill in the mechanical arts.

The "locked" state as previously described is accomplished in the present embodiment through use of a removable paddle 257, which is coupled to the sensor assembly 101 and to the first frame element 232 in the locked state. Specifically, as shown in Figs. 2b and 2c, the exemplary paddle 257 comprises a molded assembly formed from a polymer (e.g., polyethylene or ABS, for low cost and light weight yet good rigidity and other mechanical properties). The paddle 257 includes a sensor contact fork 258 disposed on its front (engagement) end 259, and a handle 260 disposed on the non-engaged end 261, the handle 260 being used to remove the paddle 257 from the apparatus 230 when unlocking the sensor assembly 101. The paddle 257 is adapted such that the fork 258 securely holds and suspends the sensor assembly 101 in a desired neutral position (i.e., with the active surface of the sensor disengaged from the subject's skin) when the paddle 257 is received within the alignment apparatus 230.

The paddle 257 include structure 259a which interfaces with complementary structure 259b formed on the first frame element 232 (see Fig. 2d) which allows the two components; i.e., paddle 257 and frame 232, to be removably coupled together via a frictional fit between the two structures 259, 259b. This arrangement allows the paddle 257 to be slidably received within the first frame 232, such that when the user/operator grasps the handle 260 and pulls in a lateral direction away from the apparatus 230, the paddle 257 (and fork 258) slide out of the frame 232, and completely disengage therefrom. The sensor is then either (i) tethered via the suspension sheet 244 if no actuator is attached, or (ii) coupled to the actuator 106 via the sensor's coupling element 104, as described in greater detail below with respect to Figs. 3-3e.

As shown most clearly in Figs. 1a and 2c, the sensor assembly 101 and paddle 257 of the present embodiment also include coupling structure 112, 264, respectively, which couples the sensor assembly 101 positively but removably to the paddle. Specifically, when the paddle 257 is inserted within the frame element 232, the coupling structures 112, 264 restrain the sensor 101 to the paddle 257, with the fork 258 of the paddle 257 supporting the sensor assembly from below. This advantageously places the sensor/actuator coupling element 104 in the desired position with respect to the first frame element 232 (and hence, with respect to the actuator arm 178 and actuator 106), thereby facilitating coupling with the actuator when the actuator 106 is mated to the arm 178 and first frame 232.

It will be further noted that in the illustrated embodiment, the presence of the paddle 257 effectively guarantees that the sensor assembly 101 (including most notably the active surface of the assembly) is completely disengaged or elevated above the surface of the skin. This advantageously allows the operator and the system itself to verify no bias of the sensor and pressure transducer during periods when such bias is undesirable, such as calibration of the sensor.

Referring now to Figs. 2e and 2f, the signal interface assembly 280 of the present embodiment of the apparatus 100 is described in detail. As shown in Fig. 2e, a first embodiment of the interface 280 comprises an electrical cable 281 having a plurality of conductors therein, the cable 281 being interposed between the sensor assembly 101 and an electrical contact element 282. Specifically, the contact element 282 is made "free floating" on the end of the cable 281, such that it can be plugged into a corresponding electrical receptacle on the actuator 106 or alternatively the parent monitoring system (not shown) and pass electrical signals between the sensor assembly 101 and the actuator/system. Such signals may include, for example electrical signals generated by the sensor (e.g., pressure transducer) during use, data relating to a storage device used in conjunction with the sensor (e.g., an EEPROM such as that described in Assignee's co-pending U.S. Patent Application Serial No. 09/652,626 filed August 31 2000 and entitled "Smart Physiologic Parameter Sensor and Method" and signals relating to the physical relationship of components in the apparatus 100 (e.g., output from the photoelectric or IR sensor(s) disposed on the actuator 106 and adapted to sense when the paddle 257 is situated properly with respect to the actuator (i.e., in the "locked" state within the frame element 232).

The contact element 282 in the illustrated embodiment comprises a substantially planar contact card 283, which includes a substrate 284 with a plurality of electrical contacts 285 formed on the surface and edges thereof, which contact corresponding contacts (not shown) in the monitoring system receptacle. Hence, the user merely slides the substrate 284 into the receptacle to form the desired electrical connections between the actuator (or parent system) and the sensor assembly 101. The sensor assembly 101 also includes a termination die 103a having contacts 288 formed thereon, the conductors of the cable 281 being terminated (e.g., soldered) to the contacts of the die 103a to form the desired electrical pathways. The terminals of the sensor element 103 are similarly electrically coupled such as via soldering to the contacts 288 of the die 103a. Any number of other electrical contact arrangements may be used within the sensor assembly, however, as will be recognized by those of ordinary skill.

The calibration and other associated data (e.g., sensor manufacturer ID data, manufacture/expiration date, patient ID, facility ID, etc.) as described in, *inter alia,* the aforementioned U.S. application Serial No. 09/652,626 is in the present embodiment stored within an EEPROM 289 disposed on the substrate 284 at the system monitoring end of the cable 281. It will be recognized, however, that the EEPROM 289 (or other storage device) may be disposed at any number of different locations, including within the sensor assembly 101. Furthermore, multiple storage devices (whether co-located or otherwise) may be utilized consistent with the invention.

It will be appreciated that the foregoing interface 280 may also be made disposable if desired by using for example low cost materials, such that the sensor assembly 101 and interface 280 can advantageously be disposed of as a unit.

The signal interface 280 of the present invention may also take on other configurations. For example, as shown in the alternative embodiment of Fig. 2f, the interface 290 comprises a flexible, substantially longitudinal lightweight substrate 291 having a narrow central section 292 and two end regions 293a, 293b. The narrow central section 292 allows for, *inter alia,* significant flexibility in both flexural and torsional dimensions. Printed conductive traces 294 are formed on/in the substrate 291 such that electrical signals can be transferred between the two end regions 293. The manufacture of low cost flexible substrates with conductive traces is well understood in the electronics arts, and accordingly not described further herein. On the first end 293a is situated the aforementioned storage device 289, in electrical communication with appropriate ones of the traces 294 and the actuator 106 via the contacts 295 formed on the substrate 291 at the first end 293a. At the second end 293b is situated the sensor 103 (e.g., pressure transducer), also electrically coupled to the appropriate traces 294. This embodiment has the advantage of very low weight and cost (due largely to the absence of a metallic conductor insulated cable), thereby reducing the resultant weight of the assessment apparatus 100 and the cost of each disposable sensor/interface assembly, respectively. Furthermore, as is well known in the art, the flexible substrate 291 of this embodiment can be made quite inexpensively if it is not designed or required to undergo a large number of flexural/torsional cycles, thereby further reducing cost. Hence, the interface device 290 of Fig. 2f allows for a significantly lower total cost for the disposable sensor/interface assembly than the embodiment of Fig. 2e previously described.

As yet another alternative embodiment of the signal interface 280, a wireless data interface (not shown) is employed. Specifically, in one embodiment, an infrared (IR) interface (such as those complying with the well known IrDA Standard) is employed to transfer signals between the sensor assembly 101 and the parent monitoring system. The IR interface obviates the need for the electrical cable 281 previously described, or any other physical data interface between the sensor assembly 101 and the parent system. Furthermore, when using the autonomous (e.g., battery powered) embodiment of the actuator 106 described below, the IR interface can also be used to transmit control data to the actuator 106, thereby obviating all cables and wires between the assessment apparatus 100 and the parent monitoring system, thereby allowing for a fully mobile solution.

In addition to or in place of the foregoing IR interface, a radio frequency (RF) interface may be utilized for passing data and/or control signals between the parent system and the apparatus 100. Such RF interfaces are well known and readily available commercially. For example, the SiW1502 Radio Modem IC manufactured by Silicon Wave Corporation of San Diego, CA, is a low-power consumption device with integrated RF logic and Bluetooth™ protocol stack adapted for Bluetooth applications. The chip is a fully integrated 2.4 GHz radio transceiver with a GFSK modem contained on a single chip. The SiW1502 chip is offered as a stand alone IC or, may be obtained with the Silicon Wave Odyssey SiW1601 Link Controller IC. The SiW1502 form factor is 7.0 x 7.0 x 1.0 mm package which is readily disposed within the interior volume of the components described herein. The Bluetooth wireless interface standard, or alternatively, other so-called "3G" (third generation) communications technologies, allows users to make wireless and instant connections between various communication devices and computers or other devices. Since Bluetooth uses radio frequency transmission, transfer of data is in real-time, and does not suffer from "line-of-sight" issues normally associated with IR interfaces.

The Bluetooth topology supports both point-to-point and point-to-multipoint connections. Multiple 'slave' devices can be set to communicate with a 'master' device. In this fashion, the assessment apparatus 100 of the present invention, when outfitted with a Bluetooth wireless suite, may communicate directly with other Bluetooth compliant mobile or fixed devices. Alternatively, a number of different subjects undergoing hemodynamic assessment according to the invention may be monitored in real time at a centralized location. For example, data for multiple different patients within the ward of a hospital undergoing hemodynamic assessment may be simultaneously monitored using a single "master" device adapted to receive and store/display the streamed data received from the various patients. A variety of other configurations are also possible.

Bluetooth-compliant devices, *inter alia,* operate in the 2.4 GHz ISM band. The ISM band is dedicated to unlicensed users, including medical facilities, thereby advantageously allowing for unrestricted spectral access by the present invention. Spectral access of the device can be accomplished via frequency divided multiple access (FDMA), frequency hopping spread spectrum (FHSS), direct sequence spread spectrum (DSSS, including code division multiple access) using a pseudo-noise spreading code, or even time division multiple access (TDMA) may be used depending on the needs of the user. For example, devices complying with IEEE Std. 802.11 may be substituted for the Bluetooth transceiver/modulator arrangement previously described if desired.

It will further be recognized that the signal interface 280 of the present invention may also comprise at least a portion of the "universal" interface circuit described in Assignee's co-pending U.S. Patent Application No. Serial No. 10/060,646 filed January 30, 2002 and entitled "Apparatus and Method for Interfacing Time-Variant Signals". Such interface circuitry advantageously permits the hemodynamic assessment apparatus 100 of the present invention to interface with most any type of parent monitor, thereby allowing for greater operational flexibility. It will be recognized that use of the aforementioned universal interface circuit (which also may disposed entirely in the parent monitoring system) advantageously extends the flexibility and scope of utility of the sensor assembly 101, interface 280, brace element 114 and actuator 106. Specifically, the universal interface circuit allows calibration (e.g., re-zeroing) of the external monitoring system without having to calibrate (re-zero) the sensor, or even know its zero value. This is to be distinguished with respect to prior art disposable pressure transducer (DPT) systems, which require calibration or re-zeroing of both the monitor and the sensor before each use. Thus, once the sensor of the present embodiment is initially zeroed, it can be interfaced to any actuator, parent monitoring system, or external patient monitor (via the universal interface circuit) without having to remove the sensor from the patient's wrist (or re-insert the paddle 257). This feature advantageously allows the caregiver to move the patient with the sensor (and brace/actuator) attached to another physical location having the same or different parent monitoring system, without obtaining any additional information regarding the sensor zero value. Thus, use of the universal interface circuit in conjunction with the apparatus 100 of the present invention effectively decouples the sensor assembly 101 from the parent system/monitor and provides the equivalent of "plug and play" capability for the sensor.

Referring now to Figs. 3-3e, one exemplary embodiment of the actuator assembly 106 of the invention is described. The actuator 106 described herein is designed to provide adjustment or movement of the position of the sensor assembly 101 in both sagittal and lateral (transverse) directions; however, it will be appreciated that it may be modified to provide more or less degrees of freedom (including, for example, proximal adjustment). Hence, the following embodiments are merely exemplary in nature.

Fig. 3 illustrates the fully assembled actuator 106 with outer case 300 and electrical interface 302, as well as signal/power interface cable 303. The outer case 300 includes an indicator 393 disposed on the upper side 305 thereof, which may be viewed by the user/operator during operation of the system. The function of this indicator 393 is described in greater detail subsequently herein.

As shown in Fig. 3a, the underside 306 of the case 300 includes the sensor drive coupling 307, as well as a coupling mechanism 308 which allows the actuator 106 to securely mate with the actuator arm 178 previously described. The coupling mechanism 308 in the present embodiment comprises a pair of diametrically opposed latches 309a, 309b (see also Fig. 3b), both of which 309 are spring-loaded and moveable such that the user can depress an un-latch button 311 on the front of the actuator 106 which compresses the spring 312 and causes the latches 309 to disengage. Specifically, both latches are spring-loaded and coupled via a toggle element that converts the motion for one latch 309a to the opposite of that for the other latch 309b. This approach allows for installation and removal of the actuator 106 from the arm 178 (and frame 232). The latches 309 also preclude the actuator 106 from rotating on the arm 178.

The underside of the actuator case 300 is also configured to include a partial bearing ring 310, which conforms substantially with the corresponding features of the first frame 232 and helps secure the actuator 106 in place to the arm 178 (and frame 232), especially under conditions of transverse loading or rotation of the actuator 106 around the lateral or proximal axes.

In the illustrated embodiment, the interface between the three components comprises having the cylindrical skirts 214 on the U-shaped arm 211 fit inside the cylindrical features 271 of the first frame 232. The partial bearing ring 310 fits around the outside of the cylindrical feature 271 of the first frame 232. It will be recognized, however, that other coupling arrangements for the actuator 106 and U-shaped arm, whether utilizing the first frame 232 or not, may be employed consistent with the invention.

As shown best in Fig. 3a, the underside of the actuator case 300 is also configured to include two ridge ports 395 adapted to receive the ridge feature 262 formed on the top surface of the paddle 257. These ports each include a sensor (described in greater detail below) used to detect the presence or absence of the paddle 257 when the actuator 106 is installed on the arm 178.

Referring now to Figs. 3c-3e, the interior components of the actuator are described. As shown in Fig. 3c, the internals of actuator 106 comprise generally a motor chassis assembly 322 with associated sensor drive coupling 307, and substrate (e.g, PCB) assembly 324. The motor chassis assembly 322 includes the hardware necessary to move the sensor drive coupling 307 in the sagittal and lateral directions, while the substrate assembly 324 contains the necessary intelligence (i.e., integrated circuits, drive circuitry, electrical terminations, discrete components, etc.) to electrically drive and control the motor chassis assembly 322, including determinations of motor position via the position encoders present in the motor chassis assembly 322. The substrate assembly 324 is generally disposed flush with and atop the motor chassis assembly 322, as shown in Fig. 3c, thereby conserving on actuator volume. The actuator internal components (including those of the motor chassis assembly 322) are advantageously disposed in a highly compact volume, an are fashioned from weight-saving materials where possible, in order to maintain the size and weight of the actuator as small as possible. This not only reduces the overall weight and size of the assessment apparatus 100 as a whole, but also allows for a smaller and lighter actuator arm 178 and supporting moveable arm 111, and even lateral positioning mechanism 136. Hence, synergistic effects resulting from the use of the present actuator 106 exist.

Referring now to Fig. 3d, the components of the motor chassis assembly 322 are shown in detail in exploded format. These components generally comprise a motor chassis frame element 340, sensor drive unit 342, applanation and lateral positioning motor (gearbox) units 343, 344 with integral position encoders 345, 346, respectively, and mechanical transmission components 348-352. As shown in Fig. 3d, the motor gearbox units 343, 344 are received substantially within the chassis frame 340, and transfer motive force to respective components of the drive unit 342 via the transmission components 348-352. Specifically, in the present embodiment, the drive unit is designed to be restrained and traverse within the chassis 340 frame under the control of the lateral positioning motor gearbox 344. Lateral positioning of the drive unit 342 (and hence sensor assembly 101) is accomplished by moving the unit 342 laterally within the chassis frame 340 along a guide shaft 397, under the motive force of the lateral positioning motor gearbox 344 via a pinion or worm gear 348, the latter driving the lateral screw gear 349, which threads through the lateral drive nut attached to the drive unit 342. Both the lateral screw gear 349 and guide shaft 397 provide support and guidance for the drive unit 342. Hence, the actuator 106 including case 300, chassis frame 340, and substrate assembly 324 remain fixed relative to the actuator arm 178, while the sensor drive unit translates laterally within the chassis 340.

The applanation motor gearbox 343 is similarly used to control the position of the sensor drive coupling 307 in the sagittal direction, albeit using different mechanisms. Specifically, as shown best in Figs. 3b and 3e, the sensor drive unit 342 includes a housing 354 containing a normally (sagittally) disposed threaded leadscrew 355, the bottom end 356 of which carries the sensor drive coupling 307. A worm gear 360 is disposed transversely (laterally) within the housing 354 and engages an internally threaded helical gear 359, the internal threads of which engage the threads of the leadscrew 355, such that when the worm gear 360 turns (under indirect motive force of the applanation motor 343, via a coupling shaft 352 which transfers the motive force to a pulley, belt 351, thereby driving the slotted shaft assembly 349), the helical gear 359 turns, and "threads" the leadscrew 355 inward or outward in the sagittal direction. The leadscrew 355 is, in the present embodiment, prevented from rotating about its longitudinal axis as it moves inward or outward by virtue of a flat region machined into a portion of the side of the leadscrew 355 along its length, which engages a comparably shaped portion of the actuator mechanism, thereby effectively restraining any rotation of the leadscrew with respect to the actuator mechanism or housing. This feature advantageously prevents the sensor assembly 101 from experiencing any rotational force or torque, which may affect any sensor readings obtained therewith.

The motor gearboxes 343, 344 used in the illustrated embodiment of Fig. 3 to drive the applanation element 102 and the lateral positioning mechanism are precision DC drive motors of the type well known in the motor arts. These motors also include one or more position encoders (not shown) which provide an electrical signal to the host system processor and associated algorithm to very precisely control the position of the applanation element (sagittally and/or laterally, as applicable) during operation. Accordingly, the variable used in the present embodiment to represent applanation element position is the number of motor increments or steps (positive or negative relative to a "zero" point); this approach advantageously removes the need to measure the absolute position with respect to the subject's tissue or anatomy. Rather, the relative number of steps is measured via the position encoder(s). This also underscores another advantage of the present apparatus; i.e., that the apparatus is "displacement" driven and therefore is controlled as a function of sensor assembly displacement, and not force. This advantageously obviates the complexities (and potential sources of error) associated with measuring force applied via a tonometric sensor or other applanation element.

It will be recognized that while DC drive motors are used in the instant embodiment, other types of motors (e.g., stepper motors, etc) may be used as the motive force for the assembly.

It will further be recognized that the exemplary embodiment of the actuator mechanism described herein allows for the separation of the movement of the sensor assembly 101 in the various directions; i.e., applanation, lateral, and proximal (not shown). Specifically, the motor chassis assembly 322 allows the leadscrew 355 to move in the normal (applanation) direction irrespective and independent of the lateral/proximal movement of the chassis assembly 322. This approach is important from the standpoint that it both allows concurrent yet independent movement in the various directions, as well as allowing for a highly compact and space/weight efficient actuator 106. Furthermore, in that a number of components within the actuator (including the motors) do not translate or dislocate within the actuator, the moving mass of the motor chassis assembly 322 is minimized, thereby reducing electrical power consumption as well as any effect on pressure measurements resulting from the translation of a mass within the actuator 106 during such measurements.

As best shown in Figs. 1a and 3a-3f, the coupling between the actuator 106 and sensor assembly 101 is accomplished using a first element 104 disposed on the sensor assembly 101 (see Fig. la) and a second corresponding element 307 mounted on the bottom of the actuator mechanism lead screw 355 (see Figs. 3a-3f). As most clearly shown in Fig. 3f, the first coupling element 104 and the second coupling element 307 are configured so as to mate together in a unitary (but readily separable) assembly when the first element is inserted within the second. In the illustrated embodiment, the first element 104 comprises a substantially pyramid-shaped and faceted dome 372 disposed atop the sensor assembly 101, including an alignment and retention feature 373 formed at the apex 374 of the dome 372. Similarly, the second element 307 attached to the actuator 106 is effectively the inverse of the first element 104; i.e., it is adapted to generally match the contours of the first element 104 and the alignment and retention feature 373 almost exactly. Hence, the first element 104 can be considered the "male" element, and the second 307 the "female" element. The substantially square shape of the base of the dome controls rotation of the first element 104 with respect to the second element 307 under torsional load. This coupling of the two elements 104, 307 allows for a highly rigid and non-compliant joint between the actuator and sensor assembly in the applanation (normal dimension), thereby effectively eliminating errors in resulting hemodynamic measurements which would arise from such compliance. This design, however, also includes enough tolerance between the coupling components to facilitate easy decoupling of the sensor assembly from the actuator, such as when the actuator 106 is removed from the arm 178. This prevents stressing or tearing of the sensor assembly 101 from the suspension sheet 244 of the alignment apparatus 230, and advantageously precludes the operator having to manually separate the sensor assembly from the actuator.

It will be noted that the pyramid shape of the elements 104, 307 further allows for coupling of the two devices under conditions of substantial misalignment; i.e., where the apex 374 of the sensor assembly dome 372 is displaced somewhat in the lateral (i.e., X-Y) plane from the corresponding recess 377 of the second element 307, and/or the sensor assembly 101 is rotated or cocked with respect to the second element 307 prior to coupling. Specifically, under such misalignment, the alignment feature 373 of the dome 372 allows the first element to slide easily within almost any portion of the interior surface area of the second element 307, such that under normal (sagittal) force, the alignment element 373 will slide into the corresponding recess 377 of the second element 307, thereby aligning the two components. This feature aids in ease of clinical operation, in that the instrument can tolerate relatively significant misalignment of the sensor and actuator (the latter due to, e.g., the actuator arm 178 not being in perfect alignment over the sensor assembly 101).

In the illustrated embodiment, while the pyramid-shaped portions of the coupling facilitate alignment of the two elements during recess, they are not relied on for mechanical strength or loading; rather, only the retention feature 373 and the base portion of the dome of the first coupling element 104 provide this functionality. This approach, while not necessary, advantageously allows for additional robustness of the device during clinical use, since foreign material and/or imperfections in the manufacturing of the first or second coupling elements (such as plastic casting "flash") can be accommodated without interfering with the coupling of the two elements, or similarly the uncoupling of the two elements when it is desired to separate the actuator from the sensor assembly. Furthermore, the contact regions of the coupling (i.e., the retention feature and the base portion) effectively transfer normal and transverse load to the sensor assembly from the actuator without requiring a tight or frictional fit, thereby further facilitating separation of the components.

It will further be recognized that while the illustrated embodiment comprises substantially pyramid-shaped elements, other shapes and sizes may be utilized with success. For example, the first and second elements 104, 307 could comprise complementary conic or frustoconical sections. As yet another alternative, a substantially spherical shape could be utilized. Other alternatives include use of multiple "domes" and/or alignment features, inversion of the first and second elements (i.e., the first element being substantially female and the second element being male), or even devices utilizing electronic sensors to aid in alignment of the two elements 104, 307.

In operation, the present embodiment of the hemodynamic assessment apparatus 100 of the invention also optionally notifies the user/operator of the presence of the sensor assembly 101 (as well as the status of its coupling to the actuator and the sufficiency of electrical tests of the sensor assembly 101) through an integrated indication. Specifically, the actuator 106 of the present embodiment includes a multi-color indicator light array 393 (in the form of a light-emitting diode) which is electrically coupled to a phototransistor which determines the presence or lack of presence of the sensor assembly 101 (specifically, the paddle 257) when the actuator 106 is installed on the actuator arm 178, and all electrical connections are made. Specifically, the presence of the sensor assembly 101 is detected by the sensing feature 262 disposed atop the paddle 257, as best shown in Fig. 2c. In the present embodiment, the LED array 393 glows yellow upon insertion of a sensor connector into the actuator 106. The system logic (e.g., software programming) then looks for the paddle 257 by determining if either pair of phototransistors have blocked optical transmission paths by virtue of the rib feature 262 of the paddle 257 being disposed into either of the ridge ports 395, thereby indicating that it is a "new" non-calibrated sensor. Specifically, calibrated sensors will have their paddle 257 removed, thereby allowing for optical transmission. If a new sensor assembly is detected, the system then "zeroes" the sensor by balancing the sensor bridge circuit and activating the LED array 393 in a selected color (e.g. green), signaling the user to remove the paddle 257. In the illustrated embodiment, the apparatus can only be calibrated with the paddle 257 in place, since the latter protects the active area at the bottom of the sensor from any loads which might affect the calibration. In addition, the EEPROM associated with the sensor assembly 101 is written with the required data to balance the sensor bridge circuit in that particular sensor.

If the installed sensor has been used before, but an intervening event has occurred (e.g., the patient has been moved), the paddle 257 will no longer be in place. In this case, the LED array 393 glows a different color (e.g., yellow) and upon insertion, the system logic would determines that the paddle 257 is not in place. The system then reads the EEPROM for the bridge circuit balancing data (previously uploaded at initial sensor use), and balances the bridge offsets. The LED array 393 is then energized to glow green. However, if the system does not detect an installed paddle 257 and cannot read the calibration data in the EEPROM, the LED array will remain yellow and an error message will optionally be displayed prompting the operator to remove the sensor assembly 101.

It will be recognized that other techniques for determining the presence of the sensor assembly 101 and/or paddle 257 may be used consistent with the invention, including mechanical switches, magnets, Hall effect sensor, infra-red, laser diodes, etc.

Additionally, other indication schemes well known to those of ordinary skill in the electronic arts may be used, including for example one or more single color LED which blinks at varying periods (including no blinking) to indicate the presence or status of the components, such as by using varying blinlc patters, sequences, and periods as error codes which the operator can use to diagnose problems, multiple LEDs, light pipes. LCD or TFT indicators, etc. The illustrated arrangement, however, has the advantages of low cost and simplicity of operator use, since the user simply waits for the green light to remove the paddle and commence measurement. Furthermore, if the red light stays illuminated, the user is alerted that a malfunction of one or more components has occurred.

In another embodiment of the apparatus 100 of the present invention, one or more accelerometers are utilized with the actuator 106 so as to provide pressure-independent motion detection for the device. As discussed in Applicant's co-owned and co-pending U.S. Patent Application Serial No. 10/211,115 entitled "Method and Apparatus for Control of Non-Invasive Parameter Measurements" filed August 1, 2002 one method for anomalous or transient signal detection involves analysis of various parameters relating to the pressure waveform, such that no external or additional sensor for motion detection is required. However, it may be desirable under certain circumstances to utilize such external or additional sensor to provide for motion detection which is completely independent of the pressure sensor and signal. Accordingly, the present embodiment includes an accelerometer (not shown) within the actuator 106 which senses motion of the actuator (and therefore the remaining components of the apparatus 100, since the two are rigidly coupled), and generates an electrical signal relating to the sensed motion. This signal is output from the actuator to the system controller/processor, and used for example to provide a windowing or gating function for the measured pressure waveform according to one or more deterministic or pre-determined threshold values. For example, when the accelerometer output signal corresponds to motion (acceleration) exceeding a given value, the controller gates the pressure waveform signal for a period of time ("deadband"), and then redetermines whether the measured acceleration still exceeds the threshold, or another reset threshold which may be higher or lower, so as to permit re-stabilization of the pressure signal. This approach avoids affects on the final calculated or displayed pressure value due to motion artifact.

Furthermore, the accelerometer(s) of the present invention can be utilized to gate or window the signal during movement of the applanation, lateral positioning, and/or proximal and distal positioning motors associated with the actuator. As will be appreciated, such movement of the motors necessarily create acceleration of the sensor assembly 101 which can affect the pressure measured by the pressure transducer used in the sensor assembly 101.

Hence, in one exemplary approach, motor movement control signals and accelerometer output act as the basis for gating the system pressure output signal, via a logical AND arrangement. Specifically, when the motor control signal and the accelerometer output (in one or more axes) are logic "high" values, the output pressure signal is blocked, with the existing displayed value preserved until the next sampling interval where valid data is present. Hence, the user advantageously sees no change in the displayed value during such gating periods. Similarly, the motors may be stopped with the trigger logic "high" values. The motors will remain stopped until the accelerometer output falls back below the threshold, and subsequently resume or restart its prescribed operation.

In another exemplary embodiment, the accelerometer operates in conjunction with the aforementioned pressure based motion detectors. The pressure based motion detectors evaluate a plurality of beats to determine whether motion has occurred and a need exists to correct for that motion. Within that detection of motion a plurality pressure signatures consistent with motion are compared against motion thresholds for starting the motion correction process. These thresholds can be adjusted (i.e. lowered to trigger more easily) when the accelerometer senses motion of the actuator.

In yet another approach, the foregoing motor control and accelerometer signals (or the accelerometer signals alone) are used for the basis for calculating and assigning a "quality" index to the pressure data, thereby indicating for example its relative weighting in any ongoing system calculations. As a simple illustration, consider where the system algorithm performs averaging of a plurality of data taken over a period of time t. Using an unweighted or non-indexed scheme, data obtained during periods of high actuator/sensor acceleration would be considered equally with those during periods or little or no acceleration. However, using the techniques of the present invention, such data taken during the high-acceleration periods may be optionally indexed such that they have less weight on the resulting calculation of the data average. Similarly, indexing as described herein can be used for more sophisticated corrections to calculations, as will be readily appreciated by those of ordinary skill in the mathematical arts. Myriad other logic and correction schemes may be used in gating or adjusting the use of sensed pressure data based at least in part on accelerometer inputs.

As will also be recognized by those of ordinary skill, a single multi-axis accelerometer device may be used consistent with the present invention, or alternatively, one or more separate devices adapted for measurement of acceleration in one axis only. For example, the ADXL202/ADXL210 "iMEMS" single-chip dual-axis IC accelerometer device manufactured by Analog Devices Inc. may be used with the actuator 106 described herein, although other devices may be substituted or used in combination.

### Methodology

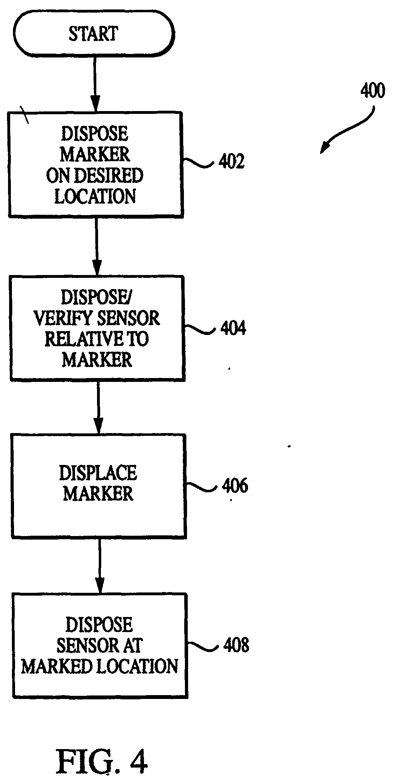

Referring now to Fig. 4, the general methodology of positioning a sensor with respect to the anatomy of the subject is described in detail. It will be recognized that while the following discussion is cast in terms of the placement of a tonometric pressure sensor (e.g., silicon strain beam device) used for measuring arterial blood pressure, the methodology is equally applicable to both other types of sensors and other parts of the subject's anatomy, human or otherwise.

As shown in Fig. 4, the illustrated embodiment of the method 400 generally comprises first disposing a marker on the location of the anatomy (step 402). In the context of the alignment apparatus 230 described above, the marker comprises the reticle 240 and alignment sheet of the second frame element 233. Specifically, in this step of the method, the user or clinician removes the backing sheet to expose the adhesive 235, and then bonds the second frame element 233 to the subject's skin, such that the reticle 240 is aligned directly over the pulse point of interest.

Next, the sensor is disposed relative to the marker if not done already (step 404). In the present context, this comprises installing or verifying that the sensor assembly 101 is installed within the first frame element 232 as previously described. In the exemplary embodiment, the first and second frame elements 232, 233 and sensor assembly 101 come "assembled" and pre-packaged, such that the user merely opens the package, removes the alignment apparatus 230 (including installed sensor assembly 101 and paddle 257), and removes the backing sheet and places the second frame element as previously described with respect to step 402.

Next, per step 406, the marker (e.g., reticle) is displaced or removed from the marked location. As previously described, this comprises in the illustrated embodiment removing the reticle via its sheet 241 from the second frame element 233. This also exposes the adhesive underlying the sheet 241.

Lastly, per step 408, the sensor assembly 101 is disposed at the desired or "marked" location (i.e., directly above the pulse point) by mating the first frame 232 to the second 233. This is accomplished in the present embodiment by actuating the fabric hinge 234 (i.e., folding the first frame onto the second via the hinge 234), such that the bottom surface of the first frame element 232 mates with the adhesive on the top surface of the second frame element 233.

While the foregoing method has been found by the Assignee hereof to have substantial benefits including ease of use and low cost, it will be recognized that any number of different combinations of these or similar steps may be used (as well as different apparatus). For example, it is feasible that the manufacturer may wish to provide the components as a kit, which the user assembles. Alternatively, the second frame element 233 may be provided separate from the first frame element 232 and sensor assembly 101 (i.e., without the hinge 234), such that the user simply places the second frame element with reticle as previously described, then removes the reticle sheet 241 thereby exposing the adhesive underneath. The first frame element 232 is then mated with the second by placing it atop the second element.

As yet another alternative, the first and second frame elements 232, 233 could be provided as a unitary assembly (with reticle); the user would then simply place the unitary frame element (not shown) using the reticle as previously described, and then mount the sensor assembly 101 thereto (after removing the reticle sheet 241) using pre-positioned mounting guides or similar structure adapted to align the sensor assembly 101 with the first frame 232, thereby inherently aligning the sensor assembly 101 to the desired pulse point.

As yet even another alternative, the aforementioned second frame element 233 may include a re-usable or attached reticle, such that for example it rotates, slides, or is otherwise dislocatable with respect to the frame element between a first position (wherein the reticle is aligned with a given point on the frame, such as where the sensor would occupy), and a second position, wherein the reticle would be displaced from interfering with the sensor assembly 101 or its movement within the frame 233 during actuation via the actuator 106.

As yet even a further alternative, the "marker" used in conjunction with the frame need not be tangible. For example, the marker may comprise a light source (such as an LED, incandescent bulb, or even low-energy laser light) which is projected onto the desired pulse point of the subject. This approach has the advantage that no physical removal of the marker is required; rather, the sensor assembly 101 can simply be swung into place over the pulse point (since the relationship of the first and second frame elements 232, 233 is predetermined), thereby interrupting the light beam with no physical interference or deleterious effects.

Alternatively, an acoustic or ultrasonic marker (or marker based on a physical parameter sensed from the subject such as pressure) can be employed. Consider the embodiment (not shown) wherein a pressure or ultrasonic sensor or array is used to precisely locate the pulse point laterally within a narrowed second frame element. The user simply places the second frame element 233 generally in the region of the desired pulse point; i.e., such that the desired pulse point is generally located within the narrow, elongated aperture formed by the frame element 233, and folds the first frame (with aforementioned sensor(s)) into position thereon. The sensor or array is then used to precisely localize the pulse point using for example a search algorithm, such as that described in Assignee's co-pending applications previously incorporated herein, to find the optimal lateral position. This advantageously obviates the need for a reticle, since the onus is on the clinician/user to place the first frame 233 properly within at least the proximal dimension. Such search method can also be extended into the proximal dimension if desired, such by including an actuator with a proximal drive motor, and a broader frame dimension.

Clearly, myriad other different combinations and configurations of the basic methodology of (i) positioning a marker with respect to a point; (ii) disposing a sensor with respect to the marker, and (iii) disposing the sensor proximate the desired point, will be recognized by those of ordinary skill given the present disclosure. The present discussion should therefore in no way be considered limiting of this broader method.

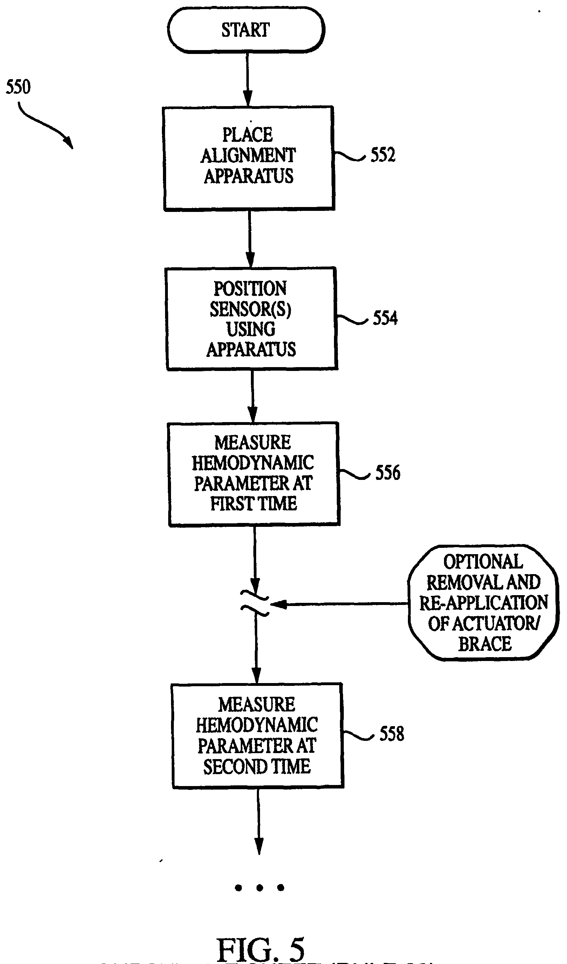

Referring now to Fig. 5, one exemplary embodiment of the improved method of recurrently measuring the blood pressure of a living subject is described. As before, the present context of the discussion is merely exemplary.

As shown in Fig. 5, the method 550 comprises first disposing an alignment apparatus adapted to align one or more sensors with respect to the anatomy of the subject (step 552). The apparatus may be the alignment apparatus 230 previously described herein, including any alternatives of forms thereof. Next, the sensor(s) is/are positioned with respect to the anatomy using the alignment apparatus (e.g., in the context of the discussion of Fig. 4, the first frame element 232 with sensor assembly 101 is folded atop the second frame 233 and adhesively bonded thereto) per step 554.

The blood pressure (or other parameter) is then measured using the sensor(s) at a first time per step 556. For example, this first measurement may occur during surgery in an operating room.

Lastly, the blood pressure or other parameter(s) of the subject are again measured using the sensor(s) at a second time subsequent to the first (step 558). Specifically, the sensor position is maintained with respect to the anatomy between measurements using the alignment apparatus 230; i.e., the frame elements 232, 233 and suspension sheet 244 cooperate to maintain the sensor assembly 101 generally atop the desired pulse point of the subject even after the actuator 106 is decoupled from the sensor 101. Herein lies a significant advantage of the present invention, in that the actuator 106 (and even the remainder of the parent hemodynamic monitoring apparatus 100, including brace 114 and adjustable arm 111) can be removed from the subject, leaving the alignment apparatus 230 in place. It may be desirable to remove the parent apparatus 100 for example where transport of the subject is desired and the present location has dedicated equipment which must remain, or the monitored subject must have the apparatus 100 removed to permit another procedure (such as post-surgical cleaning, rotation of the subject's body, etc.). Since the sensor assembly 101 is coupled to the first frame element 232 via only the suspension sheet 244 (assuming the paddle 257 is removed), and the first frame coupled to the second, the sensor assembly position is maintained effectively constant with respect to the subject pulse point where the brace 114 and actuator 106 are removed, such as during the foregoing evolutions.

Hence, when it is again desired to monitor the subject using the sensor, the brace 114 (or another similar device at the destination) is fitted to the subject, and the arm 111 adjusted such that the actuator arm 178 is coupled to the first frame element 232 of the alignment apparatus 230. The user/caregiver then merely attaches the actuator 106, which can couple to the sensor assembly 101 since the sensor assembly is still disposed in the same location with the first frame element 232 as when the first actuator was decoupled. Accordingly, no use of a second alignment apparatus or other techniques for positioning the sensor "from scratch" is needed, thereby saving time and cost. This feature further allows for more clinically significant or comparable results since the same sensor is used with effectively identical placement on the same subject; hence, and differences noted between the first and second measurements discussed above are likely not an artifact of the measurement apparatus 100.

It will be further recognized that while two measurements are described above, the alignment apparatus 230 and methodology of Fig. 4b allow for multiple such sequential decoupling-movement-recoupling events without having any significant effect on the accuracy of any measurements.

Additionally, the first and second frame elements 232, 233 can be made removably attachable such as via clips, bands, friction joints, or other types of fastening mechanisms such that the second frame element 233 can remain adhesively attached to the subject's tissue while the first frame (with sensor) is removed. The first frame 232 and sensor can then be simply re-attached to the second frame element 233 when desired. This approach reduces the mass or bulk left on the subject during transport or other procedure to an absolute minimum; i.e., only the pliable second frame element is retained on the subject's skin between measurements.

### Correction Apparatus and Methods

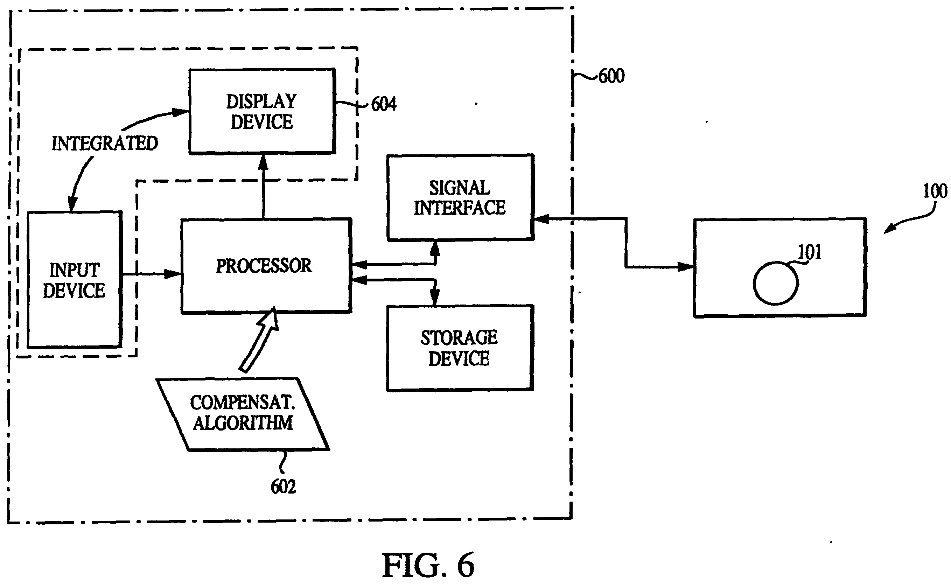

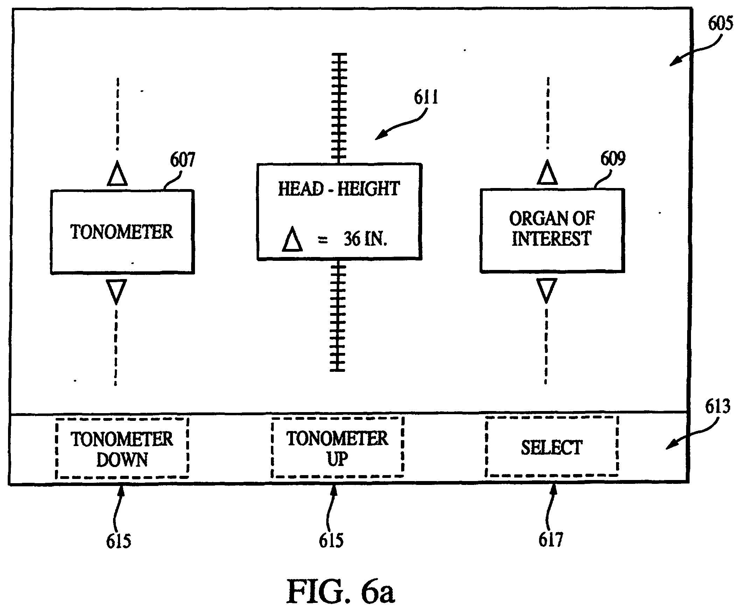

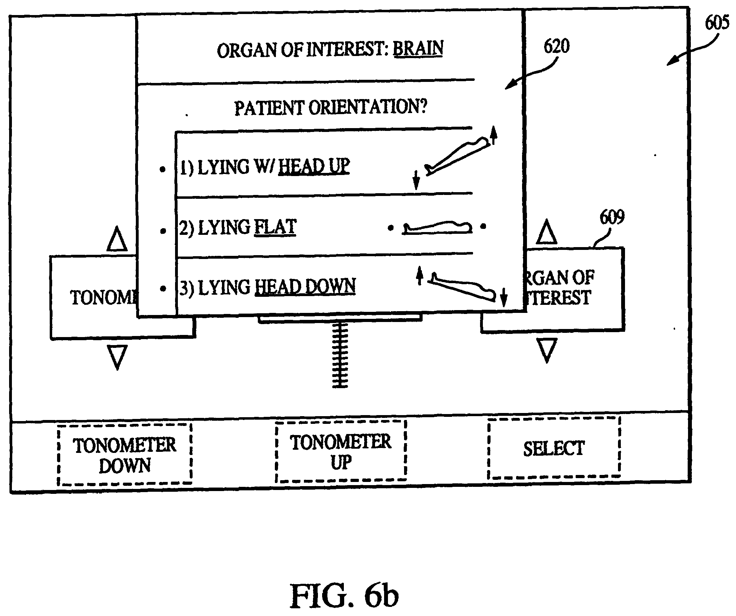

Referring now to Figs. 6-6b, another aspect of the present invention is described. This aspect of the invention contemplates the fact that the apparatus 100 previously described herein (including the sensor assembly) may reside at a different elevation during blood pressure measurement than one or more organs of interest to the caregiver, and provides a ready mechanism for compensating for such differences. Furthermore, as will be described in greater detail below, the invention may be configured to allow heuristically or even deterministically-based correction of pressure measurements for hydrodynamic effects.

As shown in the exemplary embodiment of Fig. 6, the apparatus 600 of the invention optionally includes a parametric compensation algorithm 602 adapted to allow the user to correct for hydrostatic and/or hydrodynamic effects associated with the circulatory system of the living subject. In a first exemplary embodiment, the algorithm is adapted to correct for hydrostatic effects resulting from the difference in height between the organ of interest (such as, for example, the brain) of the subject and the hemodynamic parameter (e.g., pressure) measurement location. In many situations, a significant difference between the elevations of these two locations will exist, thereby necessitating correction if a more accurate representation of pressure, etc. is to be obtained. As shown in Fig. 6a, the user is presented with a simple graphic display 605 on the display device 604 which shows a first icon 607 representing the location (elevation) of the tonometric pressure sensor, a second icon 609 representing the location of the "organ of interest", and a bar scale 611 interposed between the two icons 607, 609 which graphically illustrates the difference (Δ) in elevation between the two locations; i.e., between the pressure sensor and the organ of interest. The touch-sensitive menu 613 disposed along the bottom of the exemplary display of Fig. 6a is used to "virtually" adjust the relative position of the tonometric pressure sensor with relation to the organ of interest. Specifically, the user simply touches the regions 615 of the menu 613 labeled "tonometer down" or "tonometer up" to cause the algorithm to increase the difference in elevation for which a compensation is calculated. When a suitable differential is indicated (based on the user having a prior knowledge of the actual differential, such as for example by direct measurement), the user simply then selects the "select" function 617 on the menu 613 to enter the correction.

The foregoing display 605 is interactive, such that when the user varies the virtual position as discussed above, the icons 607, 609 move proportionately, and the displayed differential value (Δ) changes accordingly, thereby providing both a spatial and numerical representation to the user. This feature, while subtle, is significant from the standpoint that human recognition of erroneous data is often enhanced through display of a spatial indication as opposed to a purely numerical one. Much as a driver can briefly glance at their car's non-digital speedometer to determine their general speed range based solely on the position of the indicator needle, the operator of the exemplary apparatus and algorithm of Figs. 6-6a can more intuitively recognize whether an appropriate correction (i.e., one of generally the right magnitude and direction) has been applied.

Contrast the purely digital display, wherein the higher cognitive functions of the operator's brain must be engaged in order to process the data. In the aforementioned car speedometer analogy, the user must first read the displayed number, and then cognitively process this number to determine its relationship to a pre-stored (memorized) limit. Hence, the display 605 of the present embodiment advantageously mitigates the chances of applying an erroneous parametric correction, making the device clinically more robust.

This robustness may also be enhanced through the addition of other ancillary devices or algorithms to verify that the desired type and magnitude of correction is applied. For example, the software algorithms used in the system 600 may be coded with and upper "hard" limit on the magnitude of the correction which represent non-physical values, such as where a correction of that magnitude would by impossible due to human physiology. Similarly, logical checks can be employed, such as an interactive menu prompting the caregiver with questions or prompts 620 such that shown in Fig. 6b. Depending on the response entered, the system 600 will determine whether the desired correction entered via the aforementioned display 605 correlates with the entry on the menu prompt. For example, if the caregiver selects the brain as the organ of interest, and enters a negative correction via the display 605 (thereby indicating that the brain is higher in elevation than the point of pressure measurement, and that the brain pressure should be less in magnitude than that at the point of measurement), an entry on the menu 620 of Fig. 6b of "Lying flat" or "Lying with head lower" would cause the algorithm to generate an error message, and optionally prevent further measurement with the apparatus 600 until the ambiguity is resolved.

It will be recognized, however, that other display (and control) schemes may be utilized. For example, the aforementioned digital display can be used if desired. Alternatively, the digital and spatial displays can be combined, such that the display screen 605 shows both spatial and digital (alpha-numerical or symbolic) indications.

As yet another alternative, the corrections can be determined or verified automatically, such as through the use of sensors or other devices designed to determine the difference in elevation. For example, if the subject is placed in a chair or other support structure having known position and dimensions, and the anatomy of the subject constrained within certain spatial regions, the algorithm can be programmed to enter one of a plurality of predetermined corrections automatically. In an exemplary embodiment, the subject's arm is constrained to rest within a narrow band of elevation, and the subject's head is received within a contoured head rest (not shown) which is adjustable in elevation based on the subject's physical size. The elevation of the arm rest is fixed, while the head rest contains a positional sensor adapted to generate a signal in proportion to its position of adjustment for the organ of interest (i.e., brain). The compensation algorithm takes the signal from the head rest sensor, converts it to the proper format (e.g., digitizes and normalizes it), and compares it to the predetermined arm rest elevation value to derive a difference value. The difference value is then multiplied by a correction value (e.g., a hydrostatic correction) to produce a net correction in mmHg, which is then applied to all or only certain pressure measurements upon appropriate selection by the operator.

Alternatively, sensors attached to the parameter sensor (e.g., tonometric pressure sensor) and the subject's anatomy can be used to provide information regarding their relative elevations, such as through use of electromagnetic energy, electric or magnetic field intensity, acoustic energy, or other means well known in the instrumentation arts.

In yet another embodiment, the corrected (i.e., hydrostatically compensated) pressure waveform is displayed alongside or contemporaneously with the uncorrected value, the latter representing the pressure at the point of measurement.

In yet another variant, the algorithm is programmed to determine (whether via manual input or sensor signal input) the maximum correction necessary for any portion of the subject's body. In this fashion, a "bounding" or envelope curve is produced, the user knowing that the pressure associated with any organ of the subject's body will be within the indicated bounds.

With respect to hydrodynamic corrections, various schemes may be utilized for such corrections by the present invention, including (i) direct or conditioned signal input from a blood flow sensor, such as an ultrasonic transducer measuring blood flow velocity at a point upstream and/or downstream of the tonometric measurement location; (ii) a pre-stored heuristic or empirically-based correction generically applicable to all or a class of individuals; (iii) a deterministic function which determines the required hydrodynamic correction as a function of one or more input and/or sensed parameters, such as subject body mass index (BMI), cardiac output (CO), and the like; or (iv) combinations of the foregoing. In this fashion, the pressure drop induced by flow of the blood through the circulatory system of the subject can be "blacked out" to obtain a corrected representation of pressure at, for example, the aortic valve of the heart, or any other point of interest on the body.

It will also be appreciated that the algorithm of the present invention may be adapted to account for variations in the earth's gravitational field which may affect the magnitude of the hydrostatic correction applied. As is well known, the earth's gravitation field vector is not constant as a function of both elevation (altitude) and geographic position, thereby affecting the actual value of the hydrostatic pressure component, and potentially introducing further error into the pressure measurements. Such variations in the field are the result of any number of factors, including mantle density, etc. For example, a pressure measurement obtained from the same patient at high altitude at one geographic location may conceivably be different than the measurement for the identical patient (all else being equal) at a lower altitude in another geographic location, due to gravitation field variations which alter the effects of hydrostatic blood pressure. While the effects of gravitational field variation are admittedly small in magnitude, they represent yet one more variable in the measurement process which can be removed. This also has the added benefit of making the comparison of data taken from the same (or even different) patients at different geographic locations more accurate.

Note that these gravitationally-induced effects are independent of any effects of higher or lower atmospheric pressure as a function of elevation (the latter being accounted for by the apparatus 100 of the present invention through use of one or more pressure equalization ports in the sensor assembly 101).

Hence, in one exemplary embodiment, the apparatus 600 of the invention includes an algorithm adapted to determine the geographic location of the user (such as via interactive menu prompt, or even external means such as GPS satellite), and access a pre-stored database of gravitational field vectors to find the appropriate field vector for use with the aforementioned hydrostatic corrections.

In another aspect of the invention, the exemplary apparatus described herein is further optionally adapted to determine whether it is installed on the left arm or right arm of the subject, and adjust its operation accordingly. Specifically, in the case of the radial artery, the apparatus 100 determines the arm in use through detection of the position of the moving arm assembly 111 within the brace element 114. In this embodiment, the brace element 114 is made symmetric with respect to the moving arm 111 and lateral positioning mechanism 132, such that (i) either arm of the subject can be comfortably and supportedly received within the brace element 114, and (ii) the moving arm 111 can be oriented accordingly such that it is always disposed with the coupling frame 160 and associate components on the outward side of the brace (i.e., away from the subject's body). In this way, the apparatus 100 is symmetric with respect to the subject's body. Accordingly, the control algorithm associated with the apparatus 100 is made to recognize the orientation of the moving arm 111 through one or more position sensors disposed on the lateral positioning mechanism which detect the position of the frame 160 (or other components), and provide a signal to the control algorithm in order to adjust the operation of the latter, specifically to maintain the direction of sensor assembly scan during lateral positioning or other traversing operations constant with respect to the apparatus. In the present embodiment, the sensors comprise electro-optical, photodiode, or IR sensors, although other approaches may be used. For example, micro-switch or other contact arrangement may be used, or even capacitive or inductive sensing device. Myriad schemes for sensing the relative position of two components can be employed, as will be appreciated by those of ordinary skill in the art.

Alternatively, detection of the relative orientation of the components can be made manually, such as by the user entering the information (via, for example, a soft or fixed function key on the device control panel, not shown) or other means. Buttons or soft function keys labeled "left arm" and "right arm" may be used for example, or a single key/button which toggles between the allowed settings.

The primary benefit afforded by these features is consistency of measurement and removal of variables from the measurement process. Specifically, by having the control algorithm maintain a uniform direction of scan/traversal with respect to the apparatus 100, any artifacts created or existing between the various components of the apparatus and the subject's physiology are maintained constant throughout all measurements. Hence, the situation where such artifacts affect one measurement and not another is eliminated, since the artifacts will generally affect (or not affect) all measurements taken with the apparatus 100 equally.

### "Method of Providing Treatment

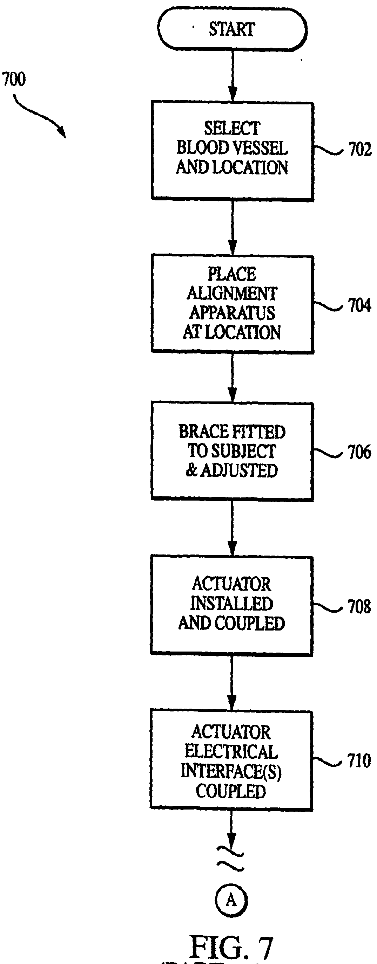

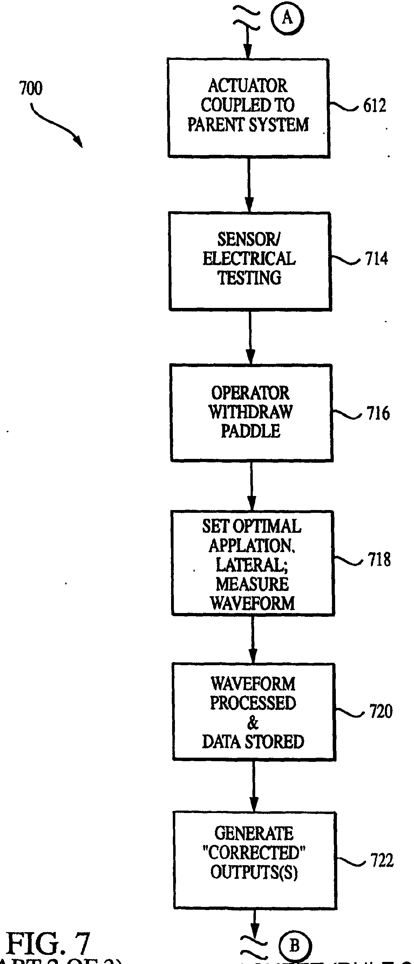

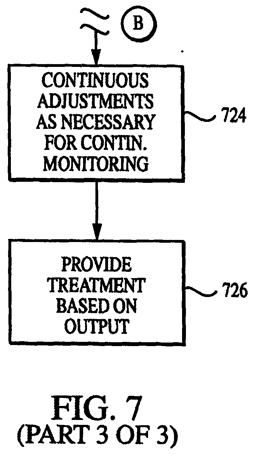

Referring now to Fig. 7, a method of providing treatment to a subject using the aforementioned methods is disclosed. As illustrated in Fig. 7, the first step 702 of the method 700 comprises selecting the blood vessel and location to be monitored. For most human subjects, this will comprise the radial artery (as monitored on the inner portion of the wrist), although other locations may be used in cases where the radial artery is compromised or otherwise not available.

Next, in step 704, the alignment apparatus 230 is placed in the proper location with respect to the subject's blood vessel, and adhered to the skin according to for example the method of Fig. 4. Such placement may be accomplished manually, i.e., by the caregiver or subject by identifying the desired pulse point (such as by feel with their finger) and visually aligning the transducer and device over the interior portion of the wrist, by the pressure/electronic/acoustic methods of positioning previously referenced, or by other means. At the conclusion of this step 704, the sensor assembly 101 is aligned above the blood vessel within the first frame element 232 with the paddle 257 installed.

Next, in step 706, the brace element 114 and associated components (i.e., adjustable arm assembly 111 with actuator arm 178) are fitted to the patient, and the various adjustments to the apparatus 100 and arm 111 made such that the U-shaped portion of the actuator arm 178 is loosely coupled (via the dowels 216 on its skirt periphery) to the corresponding elongated apertures 299 of the first frame element 232. As previously discussed, this loosely locks the two components 178, 232 together, with the elongated dimension of the apertures 299 allowing for some radial or yaw misalignment between the actuator arm 178 and the alignment apparatus 230. It also provides relative positioning of the actuator (which is coupled to the arm 178) and the sensor assembly 101 (which is coupled to the frame 232 via the paddle 257 and the suspension sheet 244).

Next, in step 708, the actuator 106 is coupled to the actuator arm 178 over the sensor as shown best in Fig. 1. The sensor assembly coupling device 104 is coupled to the actuator coupling device at the same time the actuator is mated to the arm 178, thereby completing the mechanical linlcages between the various components. Similarly, in step 710, the actuator end 283, 293 of the electrical interface 280, 290 is coupled to the actuator 106 via the port disposed on the body of the latter, and electrical continuity between the sensor assembly 101 and actuator 106 established. The fee end of the actuator cable is then connected to the parent monitoring system (step 712).

In step 714, the operation and continuity of the various devices are tested by the actuator and associated circuitry (and sensors) as previously described, and a visual indication of the results of these tests provided to the user via, e.g., the indicator LEDs 393 or similar means. Once the system electrical functions have been satisfactorily tested (including, e.g., the suitability of the sensor assembly for use on the current subject, shelf-life, etc.) and either the paddle 257 detected or the calibration data read in the EEPROM, the indicator 393 is set to "green" indicating that the paddle may be removed, and the measurements commenced.

The user then grasps the paddle 257 by its distal end and pulls outward away from the apparatus 100, thereby decoupling the sensor 101 from the paddle 257, and the paddle from the frame element 232 (step 716). The sensor assembly 101 is now "free floating" on the actuator 106, and the measurement process including any lateral positional adjustments may be performed. The optimal applanation level is also then determined as part of the measurement process. Co-pending U.S patent application Serial No. 10/072,508 previously incorporated herein illustrates one exemplary method of finding this optimum applanation level.

Once the optimal level of applanation and lateral position are set, the pressure waveform is measured per step 718, and the relevant data processed and stored as required (step 720). Such processing may include, for example, calculation of the pulse pressure (systolic minus diastolic), calculation of mean pressures or mean values over finite time intervals, and optional scaling or correction of the measured pressure waveform(s). One or more resulting outputs (e.g., systolic and diastolic pressures, pulse pressure, mean pressure, etc.) are then generated in step 722. Software processes within the parent monitoring system are then implemented as required to maintain the subject blood vessel and overlying tissue in a continuing state of optimal or near-optimal compression (as well as maintaining optimal lateral/proximal position if desired) per step 724 so as to provide continuous monitoring and evaluation of the subject's blood pressure. This is to be distinguished from the prior art techniques and apparatus, wherein only periodic representations and measurement of intra-arterial pressure are provided.

Lastly, in step 726, the "corrected" continuous measurement of the hemodynamic parameter (e.g., systolic and/or diastolic blood pressure) is used as the basis for providing treatment to the subject. For example, the corrected systolic and diastolic blood pressure values are continuously generated and displayed or otherwise provided to the health care provider in real time, such as during surgery. Alternatively, such measurements may be collected over an extended period of time and analyzed for long term trends in the condition or response of the circulatory system of the subject. Pharmacological agents or other courses of treatment may be prescribed based on the resulting blood pressure measurements, as is well known in the medical arts. Similarly, in that the present invention provides for continuous blood pressure measurement, the effects of such pharmacological agents on the subject's physiology can be monitored in real time.

It will be appreciated that the foregoing methodology of Fig. 7 may also be readily adapted to multiple hemodynamic measurements as discussed with respect to Fig. 5.

It is noted that many variations of the methods described above may be utilized consistent with the present invention. Specifically, certain steps are optional and may be performed or deleted as desired. Similarly, other steps (such as additional data sampling, processing, filtration, calibration, or mathematical analysis for example) may be added to the foregoing embodiments. Additionally, the order of performance of certain steps may be permuted, or performed in parallel (or series) if desired. Hence, the foregoing embodiments are merely illustrative of the broader methods of the invention disclosed herein.

While the above detailed description has shown, described, and pointed out novel features of the invention as applied to various embodiments, it will be understood that various omissions, substitutions, and changes in the form and details of the device or process illustrated may be made by those skilled in the art without departing from the spirit of the invention. The foregoing description is of the best mode presently contemplated of carrying out the invention. This description is in no way meant to be limiting, but rather should be taken as illustrative of the general principles of the invention. The scope of the invention should be determined with reference to the claims.

## Claims

1. Apparatus configured to position at least one sensor with respect to an anatomy, comprising:
a sensor assembly (101) comprising said at least one sensor;
two frame elements (232, 233) configured to mate in a substantially fixed relationship to one another; and
a substantially flexible suspension sheet (244) coupled to a first one of said two frame elements (232) and said at least one sensor (101), said suspension sheet (244) having
an aperture (245) in its central region through which the sensor assembly (101) is configured to mate, the suspension sheet configured to moveably and flexibly support said at least one sensor assembly with respect to said first one of said two frame elements when said at least one sensor is decoupled from a host actuator (106);
wherein said sensor assembly is configured to be coupled to said suspension sheet; and **characterized in that** a second one of said two frame elements (233) further comprises a reticle (240) and at least one feature configured to maintain said second one of said two frame elements in a constant position relative to said anatomy, said second one of said two frame elements being configured to substantially conform to said anatomy.

2. The apparatus of Claim 1, wherein said at least one sensor comprises a pressure sensor configured to sense blood pressure waveforms from a blood vessel.

3. The apparatus of Claim 1, wherein said feature of said second one of said two frame elements further comprises an adhesive element for bonding said second one of said two frame elements to skin on the surface of said anatomy.

4. The apparatus of Claim 3, wherein at least one of said two frame elements is configured to fit substantially over at least a portion of the wrist of said anatomy.

5. The apparatus of Claim 3, wherein said substantially flexible suspension sheet allows said sensor to move at least laterally and proximally with respect to at least one of said frame elements when said sensor is decoupled from said host actuator.

6. The apparatus of Claim 5, wherein said substantially flexible suspension sheet further allows said at least one sensor to move in a direction normal to the anatomy over which at least one of said frame elements is disposed so as to allow applanation at least when said at least one sensor is coupled to said host actuator.

7. The apparatus of Claim 6, wherein said substantially flexible suspension sheet further allows said at least one sensor to move in a direction normal to the anatomy over which at least one of said frame elements is disposed also when said sensor is decoupled from said host actuator.

8. The apparatus of Claim 1, wherein said substantially flexible suspension sheet allows said sensor to move in a direction normal to the anatomy over which at least one of said frame elements is disposed so as to allow applanation at least when said sensor is coupled to said host actuator.

9. The apparatus of Claim 8, wherein at least one of said frame elements is substantially flexible so as to conform to a range of different sizes of said anatomy.

10. The apparatus of Claim 1, wherein at least one of said frame elements is substantially flexible so as to conform to a range of different sizes of said anatomy.

11. The apparatus of Claim 1, wherein said sensor further comprises a silicone coating or layer disposed over at least a portion of an active face thereof

12. The apparatus of Claim 1, wherein said sensor further comprises an electrical coupling element configured to mate with an electrical interface of said host actuator.

13. The apparatus of Claim 12, wherein said sensor further comprises a raised male element configured to permit mechanical coupling with a corresponding female element disposed on said host actuator.

14. The apparatus of Claim 1, wherein at least one of said frame elements and flexible suspension sheet are fashioned from low-cost materials, thereby rendering said apparatus disposable.

## Patentansprüche

1. Ein Gerät so konfiguriert, dass zumindest ein Sensor hinsichtlich eines Körpers positioniert wird, bestehend aus:
einer Sensoreinheit (101), die zumindest einen Sensor umfasst;
zwei Rahmenelementen (232, 233),
die so konfiguriert sind, dass in wesentlich fester Beziehung miteinander verbunden sind, und
eine wesentlich flexible Suspensionsplatte (244), die mit dem ersten der genannten zwei Rahmenelemente (232)und dem genannten zumindest einen Sensor (101) verbunden ist, wobei die genannte Suspensionsplatte (244) eine Öffnung (245) in ihrem mittleren Bereich aufweist, durch welche die Sensoreinheit (101) zum Einsetzen konfiguriert wird, wobei die Suspensionsplatte so konfiguriert ist, dass sie die genannte zumindest eine Sensoreinheit hinsichtlich des ersten der genannten zwei Rahmenelemente beweglich und flexibel unterstützt, wenn der genannte zumindest eine Sensor hinsichtlich des genannten ersten der genannten zwei Rahmenelement aus einem aufnehmenden Stellglied (106) entkuppelt wird;
wobei die genannte Sensoreinheit so konfiguriert ist, dass sie mit der genannten Suspensionsplatte verbunden wird; und
**dadurch gekennzeichnet, dass** ein zweites der genannten zwei Rahmenelemente (233) zudem ein Fadenkreuz (240) und zumindest ein weiteres Merkmal aufweist, das so konfiguriert ist, dass das genannte zweite der genannten zwei Rahmenelemente in einer konstanten Position relativ zum genannten Körper gehalten wird, wobei das genannte zweite der genannten zwei Rahmenelemente so konfiguriert ist, dass es wesentlich dem genannten Körper entspricht.

2. Das Gerät entsprechend Anspruch 1, wobei der genannte zumindest eine Sensor ein Drucksensor ist, der konfiguriert ist, Blutdruckwellenformen von einem Blutgefäß zu erfassen.

3. Das Gerät entsprechend Anspruch 1, wobei das genannte Merkmal des genannten zweiten der genannten zwei Rahmenelemente zudem ein Haftelement zwecks Anhaften des genannten zweiten der genannten zwei Rahmenelemente an Haut auf der Oberfläche des genannten Körpers umfasst.

4. Das Gerät entsprechend Anspruch 3, wobei zumindest eines der genannten zwei Rahmenelemente so konfiguriert ist, dass es wesentlich zumindest über einen Teil des Handgelenks des genannten Körpers passt.

5. Das Gerät entsprechend Anspruch 3, wobei die wesentlich flexible Suspensionsplatte ermöglicht, dass der genannte Sensor sich zumindest lateral und proximal hinsichtlich zumindest einem der genannten Rahmenelemente bewegen lässt, wenn der genannte Sensor aus dem genannten aufnehmenden Stellglied entkuppelt wird.

6. Das Gerät entsprechend Anspruch 5, wobei die genannten wesentlich flexible Suspensionsplatte zudem ermöglicht, dass sich der genannte zumindest eine Sensor in einer zum Körper normalen Richtung bewegt, über dem zumindest eines der genannten Rahmenelemente so positioniert ist, dass zumindest dann Applanation möglich ist, wenn der genannte zumindest eine Sensor an das genannte aufnehmende Stellglied angeschlossen ist.

7. Das Gerät entsprechend Anspruch 6, wobei die genannten wesentlich flexible Suspensionsplatte zudem ermöglicht, dass sich der genannte zumindest eine Sensor in einer zum Körper normalen Richtung bewegt, über dem zumindest auch eines der genannten Rahmenelemente positioniert ist, wenn der genannte Sensor aus dem aufnehmende Stellglied entkuppelt wird.

8. Das Gerät entsprechend Anspruch 1, wobei die genannten wesentlich flexible Suspensionsplatte zudem ermöglicht, dass sich der genannte zumindest eine Sensor in einer zum Körper normalen Richtung bewegt, über dem zumindest eines der genannten Rahmenelemente positioniert ist, wenn der genannte Sensor in das aufnehmende Stellglied eingesetzt wird.

9. Das Gerät entsprechend Anspruch 8, wobei zumindest eines der genannten Rahmenelemente wesentlich flexibel ist, damit es einer Reihe verschiedener Größen des genannten Körpers entspricht.

10. Das Gerät entsprechend Anspruch 1, wobei zumindest eines der genannten Rahmenelemente wesentlich flexibel ist, damit es einer Reihe verschiedener Größen des genannten Körpers entspricht.

11. Das Gerät entsprechend Anspruch 1, wobei der genannte Sensor zudem eine Silikonbeschichtung oder -schicht aufweist, die sich über zumindest einen Teil einer aktiven Fläche dieses erstreckt.

12. Das Gerät entsprechend Anspruch 1, wobei der genannte Sensor zudem ein elektrisches Anschlusselement umfasst, das in eine elektrische Schnittstelle des genannten aufnehmenden Stellglied eingesetzt wird.

13. Das Gerät entsprechend Anspruch 12, wobei der genannte Sensor zudem ein hervorstehendes Steckelement umfasst, um eine mechanische Verbindung mit einem entsprechenden Buchsenelement am genannten aufnehmenden Stellglied zu ermöglichen.

14. Das Gerät entsprechend Anspruch 1, wobei zumindest entweder eines der genannten Rahmenelemente oder die flexible Suspensionsplatte aus kostengünstigen Materialien hergestellt werden, wodurch das genannte Gerät als Einweggerät verwendet werden kann.

## Revendications

1. Appareil configuré de façon à positionner au moins un capteur par rapport à une anatomie, comprenant
un ensemble capteur (101) comprenant ledit au moins un capteur ;
deux éléments bâtis (232, 233) configurés de façon à s'accoupler l'un à l'autre dans un rapport essentiellement fixe ; et
une feuille de suspension essentiellement flexible (244) rattachée à un premier desdits deux éléments bâtis (232) et audit au moins un capteur (101), ladite feuille de suspension (244) ayant une ouverture (245) dans sa région centrale à travers laquelle l'ensemble capteur (101) est configuré de façon à s'accoupler, cette feuille de suspension étant configurée de façon à supporter de façon mobile et flexible ledit au moins un ensemble capteur par rapport audit premier desdits deux éléments bâtis lorsque ledit premier desdits deux capteurs est détaché d'un actionneur hôte (106) ;
dans lequel ledit ensemble capteur est configuré de façon à être rattaché à ladite feuille de suspension ; et
**caractérisé en ce qu'**un deuxième desdits deux éléments bâtis (233) comporte en outre un réticule (240) et au moins une caractéristique configurée de façon à maintenir ledit deuxième desdits deux éléments bâtis dans une position constante par rapport à ladite anatomie, ledit deuxième desdits deux éléments bâtis étant configuré de façon à se conformer essentiellement à ladite anatomie.

2. Appareil selon la revendication 1, dans lequel ledit au moins un capteur consiste en un capteur de pression configuré de façon à détecter les formes d'onde de pression artérielle à partir d'un vaisseau sanguin.

3. Appareil selon la revendication 1, dans lequel ladite caractéristique dudit deuxième desdits deux éléments bâtis comprend en outre un élément adhésif pour coller ledit deuxième desdits deux éléments bâtis sur la peau sur la surface de ladite anatomie.

4. Appareil selon la revendication 3, dans lequel au moins un desdits deux éléments bâtis est configuré de façon à s'ajuster essentiellement sur au moins une partie du poignet de ladite anatomie.

5. Appareil selon la revendication 3, dans lequel ladite feuille de suspension essentiellement flexible permet audit capteur de bouger au moins latéralement et de façon proximale par rapport à au moins un desdits éléments bâtis lorsque ledit capteur est détaché dudit actionneur hôte.

6. Appareil selon la revendication 5, dans lequel ladite feuille de suspension essentiellement flexible permet en outre audit au moins un capteur de bouger dans une direction normale à l'anatomie au-dessus de laquelle au moins un desdits éléments bâtis est disposé de façon à permettre une aplanation au moins lorsque ledit au moins un capteur est rattaché audit actionneur hôte.

7. Appareil selon la revendication 6, dans lequel ladite feuille de suspension essentiellement flexible permet en outre audit au moins un capteur de bouger dans une direction normale à l'anatomie au-dessus de laquelle au moins un desdits éléments bâtis est disposé aussi lorsque ledit au moins un capteur est détaché dudit actionneur hôte.

8. Appareil selon la revendication 1, dans lequel ladite feuille de suspension essentiellement flexible permet audit capteur de bouger dans une direction normale à l'anatomie au-dessus de laquelle au moins un desdits éléments bâtis est disposé de façon à permettre une aplanation au moins lorsque ledit capteur est rattaché audit actionneur hôte.

9. Appareil selon la revendication 8, dans lequel au moins un desdits éléments bâtis est essentiellement flexible de façon à se conformer à un gamme de différentes tailles de ladite anatomie.

10. Appareil selon la revendication 1, dans lequel au moins un desdits éléments bâtis est essentiellement flexible de façon à se conformer à un gamme de différentes tailles de ladite anatomie.

11. Appareil selon la revendication 1, dans lequel ledit capteur comporte en outre un revêtement ou une couche de silicone disposée au-dessus d'au moins une partie d'une face active de celui-ci.

12. Appareil selon la revendication 1, dans lequel ledit capteur comporte en outre un élément de couplage électrique configuré de façon à s'accoupler avec une interface électrique dudit actionneur hôte.

13. Appareil selon la revendication 12, dans lequel ledit capteur comporte en outre un élément mâle surélevé configuré de façon à permettre l'accouplement mécanique avec un élément femelle correspondant disposé sur ledit actionneur hôte.

14. Appareil selon la revendication 1, dans lequel au moins un desdits éléments bâtis et de la feuille de suspension flexible sont fabriqués à partir de matériaux peu coûteux, rendant ledit appareil jetable.
